# EUROPEAN PATENT SPECIFICATION

(11) **EP 1 673 393 B1**
(45) Date of publication and mention of the grant of the patent: **08.04.2009**
(21) Application number: 04775280.3
(22) Date of filing: 24.09.2004
(51) Int. Cl.: C07K 16/00, C07K 16/08, C07K 16/44, C07K 16/40, A61K 39/385, G01N 33/574, G01N 33/53

(54) **METHODS, KITS, AND COMPOSITIONS FOR THE DEVELOPMENT AND USE OF MONOCLONAL ANTIBODIES SPECIFIC TO ANTIGENS OF LOW IMMUNOGENICITY**
VERFAHREN, KITS UND ZUSAMMENSETZUNGEN ZUR ENTWICKLUNG UND ANWENDUNG VON FÜR ANTIGENE NIEDRIGER IMMUNOGENIZITÄT SPEZIFISCHEN MONOKLONALEN ANTIKÖRPERN
METHODES, TROUSSES ET COMPOSITIONS PERMETTANT DE DEVELOPPER ET D'UTILISER DES ANTICORPS MONOCLONAUX SPECIFIQUES D'ANTIGENES PRESENTANT HABITUELLEMENT UNE FAIBLE ANTIGENICITE

(30) Priority: 25.09.2003 RU 2003128660
(43) Date of publication of application: 28.06.2006
(73) Proprietor: Kiselev, Vsevolod Ivanovich, Moscow, 113149 (RU); Sveshnikov, Petr Georgiyevich, Moscow, 117639 (RU)
(72) Inventor: Kiselev, Vsevolod Ivanovich, Moscow, 113149 (RU); Sveshnikov, Petr Georgiyevich, Moscow, 117639 (RU)
(74) Representative: Williams, Aylsa
(86) International application number: PCT/RU2004/000373
(87) International publication number: WO 2005/028510

(56) References cited:
- EP-A- 0 299 354
- WO-A-00/65357
- WO-A-00/78344
- WO-A-02/092131
- DE-A1- 4 224 542
- PATENT ABSTRACTS OF JAPAN vol. 1997, no. 09, 30 September 1997 (1997-09-30) & JP 09 132600 A (FUJI YAKUHIN KOGYO KK), 20 May 1997 (1997-05-20)
- SEEDORF K ET AL: "IDENTIFICATION OF EARLY PROTEINS OF THE HUMAN PAPILLOMA VIRUSES TYPE 16 (HPV 16) AND TYPE 18 (HPV 18) IN CERVICAL CARCINOMA CELLS" EMBO JOURNAL, OXFORD UNIVERSITY PRESS, SURREY, GB, vol. 6, no. 1, 1987, pages 139-144, XP001021907 ISSN: 0261-4189
- KOLLER MICHAEL F ET AL: "Induction of antibodies against murine full-length prion protein in wild-type mice." JOURNAL OF NEUROIMMUNOLOGY, vol. 132, no. 1-2, November 2002 (2002-11), pages 113-116, XP002316821 ISSN: 0165-5728

### SEQUENCE LISTING

<110> Kiselev, Vsevolod I
   Petr, Sveshnikov G
<120> METHODS, KITS, AND COMPOSITIONS FOR THE DEVELOPMENT AND USE OF MONOCLONAL ANTIBODIES SPECIFIC TO ANTIGENS TRADITIONALLY OF LOW IMMUNOGENICITY
<130> Immunize
<150> RU 2003128660
   <151> 2003-09-25
<160> 22
<170> PatentIn version 3.1
<210> 1
   <211> 309
   <212> DNA
   <213> Human papillomavirus type 16
<220>
   <221> CDS
   <222> (7)..(303)
   <223>
<400> 1
   gaattc atc atg cat gga gat aca cct aca ttg cat gaa tat atg tta 48
   Ile Met His Gly Asp Thr Pro Thr Leu His Glu Tyr Met Leu
   1 5 10
   gat ttg caa cca gag aca act gat ctc tac tgt tat gag caa tta aat 96
   Asp Leu Gln Pro Glu Thr Thr Asp Leu Tyr Cys Tyr Glu Gln Leu Asn
   15 20 25 30
   gac agc tca gag gag gag gat gaa ata gat ggt cca gct gga caa gca 144
   Asp Ser Ser Glu Glu Glu Asp Glu Ile Asp Gly Pro Ala Gly Gln Ala
   35 40 45
   gaa ccg gac aga gcc cat tac aat att gta acc ttt tgt tgc aag tgt 192
   Glu Pro Asp Arg Ala His Tyr Asn Ile Val Thr Phe Cys Cys Lys Cys
   50 55 60
   gac tct acg ctt cgg ttg tgc gta caa agc aca cac gta gac att cgt 240
   Asp Ser Thr Leu Arg Leu Cys Val Gln Ser Thr His Val Asp Ile Arg
   65 70 75
   act ttg gaa gac ctg tta atg ggc aca cta gga att gtg tgc ccc atc 288
   Thr Leu Glu Asp Leu Leu Met Gly Thr Leu Gly Ile Val Cys Pro Ile
   80 85 90
   tgt tct cag aaa cca ggatcc 309
   Cys Ser Gln Lys Pro
   95
<210> 2
   <211> 99
   <212> PRT
   <213> Human papillomavirus type 16
<400> 2
   Ile Met His Gly Asp Thr Pro Thr Leu His Glu Tyr Met Leu Asp Leu
   1 5 10 15
   Gln Pro Glu Thr Thr Asp Leu Tyr Cys Tyr Glu Gln Leu Asn Asp Ser
   20 25 30
   Ser Glu Glu Glu Asp Glu Ile Asp Gly Pro Ala Gly Gln Ala Glu Pro
   35 40 45
   Asp Arg Ala His Tyr Asn Ile Val Thr Phe Cys Cys Lys Cys Asp Ser
   50 55 60
   Thr Leu Arg Leu Cys Val Gln Ser Thr His Val Asp Ile Arg Thr Leu
   65 70 75 80
   Glu Asp Leu Leu Met Gly Thr Leu Gly Ile Val Cys Pro Ile Cys Ser
   85 90 95
   Gln Lys Pro
<210> 3
   <211> 330
   <212> DNA
   <213> Human papillomavirus type 18
<220>
   <221> CDS
   <222> (7)..(324)
   <223>
<400> 3
   gaattc agt atg cat gga cct aag gca aca ttg caa gac att gta ttg 48
   Ser Met His Gly Pro Lys Ala Thr Leu Gln Asp Ile Val Leu
   1 5 10
   cat tta gag ccc caa aat gaa att ccg gtt gac ctt cta tgt cac gag 96
   His Leu Glu Pro Gln Asn Glu Ile Pro Val Asp Leu Leu Cys His Glu
   15 20 25 30
   caa tta agc gac tca gag gaa gaa aac gat gaa ata gat gga gtt aat 144
   Gln Leu Ser Asp Ser Glu Glu Glu Asn Asp Glu Ile Asp Gly Val Asn
   35 40 45
   cat caa cat tta cca gcc cga cga gct gaa cca caa cgt cac aca atg 192
   His Gln His Leu Pro Ala Arg Arg Ala Glu Pro Gln Arg His Thr Met
   50 55 60
   ttg tgt atg tgt tgt aag tgt gaa gcc aga att gag cta gta gta gaa 240
   Leu Cys Met Cys Cys Lys Cys Glu Ala Arg Ile Glu Leu Val Val Glu
   65 70 75
   agc tca gca gac gac ctt cga gca ttc cag cag ctg ttt ctg aac acc 288
   Ser Ser Ala Asp Asp Leu Arg Ala Phe Gln Gln Leu Phe Leu Asn Thr
   80 85 90
   ctg tcc ttt gtg tgt ccg tgg tgt gca tcc cag cag ggatcc 330
   Leu Ser Phe Val Cys Pro Trp Cys Ala Ser Gln Gln
   95 100 105
<210> 4
   <211> 106
   <212> PRT
   <213> Human papillomavirus type 18
<400> 4
   Ser Met His Gly Pro Lys Ala Thr Leu Gln Asp Ile Val Leu His Leu
   1 5 10 15
   Glu Pro Gln Asn Glu Ile Pro Val Asp Leu Leu Cys His Glu Gln Leu
   20 25 30
   Ser Asp Ser Glu Glu Glu Asn Asp Glu Ile Asp Gly Val Asn His Gln
   35 40 45
   His Leu Pro Ala Arg Arg Ala Glu Pro Gln Arg His Thr Met Leu Cys
   50 55 60
   Met Cys Cys Lys Cys Glu Ala Arg Ile Glu Leu Val Val Glu Ser Ser
   65 70 75 80
   Ala Asp Asp Leu Arg Ala Phe Gln Gln Leu Phe Leu Asn Thr Leu Ser
   85 90 95
   Phe Val Cys Pro Trp Cys Ala Ser Gln Gln
   100 105
<210> 5
   <211> 5321
   <212> DNA
   <213> Artificial Sequence
<220>
   <223> Nucleotide sequence of recombinant vector pQE30-dnaK
<400> 5
   ctcgagaaat cataaaaaat ttatttgctt tgtgagcgga taacaattat aatagattca 60
   attgtgagcg gataacaatt tcacacagaa ttcattaaag aggagaaatt aactatgaga 120
   ggatcgcatc accatcacca tcacggatcc gctcgtgcgg tcgggatcga cctcgggacc 180
   accaactccg tcgtctcggt tctggaaggt ggcgacccgg tcgtcgtcgc caactccgag 240
   ggctccagga ccaccccgtc aattgtcgcg ttcgcccgca acggtgaggt gctggtcggc 300
   cagcccgcca agaaccaggc agtgaccaac gtcgatcgca ccgtgcgctc ggtcaagcga 360
   cacatgggca gcgactggtc catagagatt gacggcaaga aatacaccgc gccggagatc 420
   agcgcccgca ttctgatgaa gctgaagcgc gacgccgagg cctacctcgg tgaggacatt 480
   accgacgcgg ttatcacgac gcccgcctac ttcaatgacg cccagcgtca ggccaccaag 540
   gacgccggcc agatcgccgg cctcaacgtg ctgcggatcg tcaacgagcc gaccgcggcc 600
   gcgctggcct acggcctcga caagggcgag aaggagcagc gaatcctggt cttcgacttg 660
   ggtggtggca ctttcgacgt ttccctgctg gagatcggcg agggtgtggt tgaggtccgt 720
   gccacttcgg gtgacaacca cctcggcggc gacgactggg accagcgggt cgtcgattgg 780
   ctggtggaca agttcaaggg caccagcggc atcgatctga ccaaggacaa gatggcgatg 840
   cagcggctgc gggaagccgc cgagaaggca aagatcgagc tgagttcgag tcagtccacc 900
   tcgatcaacc tgccctacat caccgtcgac gccgacaaga acccgttgtt cttagacgag 960
   cagctgaccc gcgcggagtt ccaacggatc actcaggacc tgctggaccg cactcgcaag 1020
   ccgttccagt cggtgatcgc tgacaccggc atttcggtgt cggagatcga tcacgttgtg 1080
   ctcgtgggtg gttcgacccg gatgcccgcg gtgaccgatc tggtcaagga actcaccggc 1140
   ggcaaggaac ccaacaaggg cgtcaacccc gatgaggttg tcgcggtggg agccgctctg 1200
   caggccggcg tcctcaaggg cgaggtgaaa gacgttctgc tgcttgatgt taccccgctg 1260
   agcctgggta tcgagaccaa gggcggggtg atgaccaggc tcatcgagcg caacaccacg 1320
   atccccacca agcggtcgga gactttcacc accgccgacg acaaccaacc gtcggtgcag 1380
   atccaggtct atcaggggga gcgtgagatc gccgcgcaca acaagttgct cgggtccttc 1440
   gagctgaccg gcatcccgcc ggcgccgcgg gggattccgc agatcgaggt cactttcgac 1500
   atcgacgcca acggcattgt gcacgtcacc gccaaggaca agggcaccgg caaggagaac 1560
   acgatccgaa tccaggaagg ctcgggcctg tccaaggaag acattgaccg catgatcaag 1620
   gacgccgaag cgcacgccga ggaggatcgc aagcgtcgcg aggaggccga tgttcgtaat 1680
   caagccgaga cattggtcta ccagacggag aagttcgtca aagaacagcg tgaggccgag 1740
   ggtggttcga aggtacctga agacacgctg aacaaggttg atgccgcggt ggcggaagcg 1800
   aaggcggcac ttggcggatc ggatatttcg gccatcaagt cggcgatgga gaagctgggc 1860
   caggagtcgc aggctctggg gcaagcgatc tacgaagcag ctcaggctgc gtcacaggcc 1920
   actggcgctg cccaccccgg cggcgagccg ggcggtgccc accccggctc ggctgatgac 1980
   gttgtggacg cggaggtggt cgacgacggc cgggaggcca agtgacggac gggtcgacct 2040
   gcagccaagc ttaattagct gagcttggac tcctgttgat agatccagta atgacctcag 2100
   aactccatct ggatttgttc agaacgctcg gttgccgccg ggcgtttttt attggtgaga 2160
   atccaagcta gcttggcgag attttcagga gctaaggaag ctaaaatgga gaaaaaaatc 2220
   actggatata ccaccgttga tatatcccaa tggcatcgta aagaacattt tgaggcattt 2280
   cagtcagttg ctcaatgtac ctataaccag accgttcagc tggatattac ggccttttta 2340
   aagaccgtaa agaaaaataa gcacaagttt tatccggcct ttattcacat tcttgcccgc 2400
   ctgatgaatg ctcatccgga atttcgtatg gcaatgaaag acggtgagct ggtgatatgg 2460
   gatagtgttc acccttgtta caccgttttc catgagcaaa ctgaaacgtt ttcatcgctc 2520
   tggagtgaat accacgacga tttccggcag tttctacaca tatattcgca agatgtggcg 2580
   tgttacggtg aaaacctggc ctatttccct aaagggttta ttgagaatat gtttttcgtc 2640
   tcagccaatc cctgggtgag tttcaccagt tttgatttaa acgtggccaa tatggacaac 2700
   ttcttcgccc ccgttttcac catgggcaaa tattatacgc aaggcgacaa ggtgctgatg 2760
   ccgctggcga ttcaggttca tcatgccgtt tgtgatggct tccatgtcgg cagaatgctt 2820
   aatgaattac aacagtactg cgatgagtgg cagggcgggg cgtaattttt ttaaggcagt 2880
   tattggtgcc cttaaacgcc tggggtaatg actctctagc ttgaggcatc aaataaaacg 2940
   aaaggctcag tcgaaagact gggcctttcg ttttatctgt tgtttgtcgg tgaacgctct 3000
   cctgagtagg acaaatccgc cctctagagc tgcctcgcgc gtttcggtga tgacggtgaa 3060
   aacctctgac acatgcagct cccggagacg gtcacagctt gtctgtaagc ggatgccggg 3120
   agcagacaag cccgtcaggg cgcgtcagcg ggtgttggcg ggtgtcgggg cgcagccatg 3180
   acccagtcac gtagcgatag cggagtgtat actggcttaa ctatgcggca tcagagcaga 3240
   ttgtactgag agtgcaccat atgcggtgtg aaataccgca cagatgcgta aggagaaaat 3300
   accgcatcag gcgctcttcc gcttcctcgc tcactgactc gctgcgctcg gtcggtcggc 3360
   tgcggcgagc ggtatcagct cactcaaagg cggtaatacg gttatccaca gaatcagggg 3420
   ataacgcagg aaagaacatg tgagcaaaag gccagcaaaa ggccaggaac cgtaaaaagg 3480
   ccgcgttgct ggcgtttttc cataggctcc gcccccctga cgagcatcac aaaaatcgac 3540
   gctcaagtca gaggtggcga aacccgacag gactataaag ataccaggcg tttccccctg 3600
   gaagctccct cgtgcgctct cctgttccga ccctgccgct taccggatac ctgtccgcct 3660
   ttctcccttc gggaagcgtg gcgctttctc atagctcacg ctgtaggtat ctcagttcgg 3720
   tgtaggtcgt tcgctccaag ctgggctgtg tgcacgaacc ccccgttcag cccgaccgct 3780
   gcgccttatc cggtaactat cgtcttgagt ccaacccggt aagacacgac ttatcgccac 3840
   tggcagcagc cactggtaac aggattagca gagcgaggta tgtaggcggt gctacagagt 3900
   tcttgaagtg gtggcctaac tacggctaca ctagaaggac agtatttggt atctgcgctc 3960
   tgctgaagcc agttaccttc ggaaaaagag ttggtagctc ttgatccggc aaacaaacca 4020
   ccgctggtag cggtggtttt tttgtttgca agcagcagat tacgcgcaga aaaaaaggat 4080
   ctcaagaaga tcctttgatc ttttctacgg ggtctgacgc tcagtggaac gaaaactcac 4140
   gttaagggat tttggtcatg agattatcaa aaaggatctt cacctagatc cttttaaatt 4200
   aaaaatgaag ttttaaatca atctaaagta tatatgagta aacttggtct gacagttacc 4260
   aatgcttaat cagtgaggca cctatctcag cgatctgtct atttcgttca tccatagttg 4320
   cctgactccc cgtcgtgtag ataactacga tacgggaggg cttaccatct ggccccagtg 4380
   ctgcaatgat accgcgagac ccacgctcac cggctccaga tttatcagca ataaaccagc 4440
   cagccggaag ggccgagcgc agaagtggtc ctgcaacttt atccgcctcc atccagtcta 4500
   ttaattgttg ccgggaagct agagtaagta gttcgccagt taatagtttg cgcaacgttg 4560
   ttgccattgc tacaggcatc gtggtgtcac gctcgtcgtt tggtatggct tcattcagct 4620
   ccggttccca acgatcaagg cgagttacat gatcccccat gttgtgcaaa aaagcggtta 4680
   gctccttcgg tcctccgatc gttgtcagaa gtaagttggc cgcagtgtta tcactcatgg 4740
   ttatggcagc actgcataat tctcttactg tcatgccatc cgtaagatgc ttttctgtga 4800
   ctggtgagta ctcaaccaag tcattctgag aatagtgtat gcggcgaccg agttgctctt 4860
   gcccggcgtc aatacgggat aataccgcgc cacatagcag aactttaaaa gtgctcatca 4920
   ttggaaaacg ttcttcgggg cgaaaactct caaggatctt accgctgttg agatccagtt 4980
   cgatgtaacc cactcgtgca cccaactgat cttcagcatc ttttactttc accagcgttt 5040
   ctgggtgagc aaaaacagga aggcaaaatg ccgcaaaaaa gggaataagg gcgacacgga 5100
   aatgttgaat actcatactc ttcctttttc aatattattg aagcatttat cagggttatt 5160
   gtctcatgag cggatacata tttgaatgta tttagaaaaa taaacaaata ggggttccgc 5220
   gcacatttcc ccgaaaagtg ccacctgacg tctaagaaac cattattatc atgacattaa 5280
   cctataaaaa taggcgtatc acgaggccct ttcgtcttca c 5321
<210> 6
   <211> 12
   <212> PRT
   <213> Bos taurus
<400> 6
   Lys Lys Arg Pro Lys Pro Gly Gly Gly Trp Asn Thr
   1 5 10
<210> 7
   <211> 8
   <212> PRT
   <213> bos taurus
<400> 7
   Gln Pro His Gly Gly Gly Trp Gly
   1 5
<210> 8
   <211> 13
   <212> PRT
   <213> bos taurus
<400> 8
   Gln Trp Asn Lys Pro Ser Lys Pro Lys Thr Asn Ile Lys
   1 5 10
<210> 9
   <211> 17
   <212> PRT
   <213> bos taurus
<400> 9
   Ile Thr Gln Tyr Gln Arg Glu Ser Gln Ala Tyr Tyr Gln Arg Gly Ala
   1 5 10 15
   Ser
<210> 10
   <211> 19
   <212> DNA
   <213> Human papillomavirus type 16
<400> 10
   tgacagctca gaggaggag 19
<210> 11
   <211> 19
   <212> DNA
   <213> Human papillomavirus type 16
<400> 11
   gcacaaccga agcgtagag 19
<210> 12
   <211> 20
   <212> DNA
   <213> Human papillomavirus type 18
<400> 12
   gcgactcaga ggaagaaaac 20
<210> 13
   <211> 20
   <212> DNA
   <213> Human papillomavirus type 18
<400> 13
   caaaggacag ggtgttcaga 20
<210> 14
   <211> 31
   <212> DNA
   <213> Human papillomavirus type 18
<400> 14
   tctaacgaat tcagtatgca tggacctaag g 31
<210> 15
   <211> 30
   <212> DNA
   <213> Human papillomavirus type 18
<400> 15
   attacaggat ccctgctggg atgcacacca 30
<210> 16
   <211> 31
   <212> DNA
   <213> Human papillomavirus type 16
<400> 16
   attctcgaat tcatcatgca tggagataca c 31
<210> 17
   <211> 31
   <212> DNA
   <213> Human papillomavirus type 16
<400> 17
   cttatcggat cctggtttct gagaacagat g 31
<210> 18
   <211> 130
   <212> DNA
   <213> Artificial sequence
<220>
   <223> pHE716 and pHE718 terminal sequences
<220>
   <221> misc_feature
   <222> (107)..(108)
   <223> HSP 16/HSP18 E7 gene insertion site
<400> 18
   taatacgact cactataggg agaccacaac ggtttccctc tagaaataat tttgtttaac 60
   tttaagaagg agatatacat atgcatcacc atcaccatca cgaattcgga tcctaattag 120
   ctgaaagctt 130
<210> 19
   <211> 28
   <212> DNA
   <213> Artificial sequence
<220>
   <223> Forward primer for pHE716
<400> 19
   gaagatctat gcatggagat acacctac 28
<210> 20
   <211> 28
   <212> DNA
   <213> Artificial Sequence
<220>
   <223> reverse primer for pHE716
<400> 20
   cgggatcctg gtttctgaga acagatgg 28
<210> 21
   <211> 28
   <212> DNA
   <213> Artificial Sequence
<220>
   <223> Forward primer for pHE718
<400> 21
   gaagatctat gcatggacct aaggcaac 28
<210> 22
   <211> 28
   <212> DNA
   <213> Artificial Sequence
<220>
   <223> Reverse primer for pHE718
<400> 22
   cgggatccct gctgggatgc acaccacg 28

## Description

### Field of the Invention

The methods and compositions of the invention are in the field of medical biochemistry and relates generally to the development and use of monoclonal antibodies specific to antigens traditionally of low immunogenicity.

### Background of the invention

The immune system has two arms, the adaptive and innate immune responses. These two arms of the immune system work together to combat a foreign invader. Any substance capable of eliciting an adaptive immune response is referred to as an antigen. Foreign molecules can act as antigens and stimulate an immune response resulting in the production of antibodies. Some molecules, however, do not stimulate an immune response. In the past, this was overcome by using an adjuvant, like Freund's complete adjuvant, in order to activate the innate immune system.

Despite the use of adjuvants, there still remain many molecules, however, that do not elicit an immune response resulting in the production of antigen-specific antibodies. Particularly, many antigens from natural sources often do not elicit a sufficient and molecule-specific immune response. In many instances, chemical synthesis and recombinant technology is used to produce the antigen. These synthetically obtained antigens, however, often do not have the same tertiary structure as the native molecule. When antibodies are developed to the synthetic antigens, the antibodies do not recognize the native molecule.
EP 0 299 354 teaches monoclonal antibodies against E7 protein of human type 16 papillomavirus, a process for their preparation and their use.
DE 42 24 542 describes monoclonal antibodies that are produced by immunizing a mouse with a conjugate of a major histocompatibility complex class I molecule and a peptide antigen.
Patent Abstract of Japan Vol. 1997, No.09 30 September 1997 relates to a monoclonal antibody immunoreactive specifically with rabbit matrix metalloprotease (MMP-3) obtained by immunizing the mouse with rabbit MMP-3 bound to a carrier protein such as bovine serum album.
WO00/65357 teaches a method of pre-clinical and clinical diagnosis of transmissible spongiform encephalopathies, wherein the altered expression of a marker protein is measured. This document teaches a test kit using antibodies specific to the marker protein.

The present invention provides novel methods and compositions that allow the production of antigen-specific antibodies to antigens that have traditionally been unable to elicit an adequate and specific immune response. These antibodies are useful for many different applications such as, but not limited to, therapies for disease (such as, but not limited to, cancer, viral infections, etc.), immunodiagnostics, immunochemistry, immunohistology, immunocytology, immunoaffinity chromatography, and genomic and proteomic research.

### Summary of the invention

The present invention relates to methods of producing monoclonal antibodies specific to an antigen of low immunogencity comprising conjugating the antigen chemically to a carrier molecule, wherein the carrier molecule is a heat-shock protein; immunizing an animal with the conjugated antigen; harvesting B cells from the animal; creating a hybridomas from the harvested B cells; and screening the hybridomas for specificity to the native antigen. In one embodiment, the carrier molecule is HSP70. In another embodiment, the animal has an intact immune system. In yet another embodiment, the animal is a mammal. In further embodiments, B cells are harvested from ascites, spleen, lymph nodes, or blood, either individually or in combination, in yet further embodiments, the hybridoma is created using an immortal cell, such as, but not limited to a mouse myeloma cell, an immortal human cell, or an immortal rat cell. In other embodiments, the screening for specificity is done by a method chosen from the group consisting of radioimmunoassay, enzyme-linked immunosorbant assay, "sandwich" immunoassay, immunoradiometric assay, gel diffusion precipitation reaction, immunodiffusion assay, *in situ* immunoassay, western blot, precipitation reaction, agglutination assay, complement fixation assay, immunofluorescence assay, protein A assay, virus visualization assay, biological activity modulation asay, and immunoelectrophoresis assay.

The present invention also relates to compositions comprising a monoclonal antibody specific to an antigen of low immunogenicity produced by conjugating the antigen chemically to a carrier molecule wherein the carrier molecule is a heat-shock protein, immunizing an animal with the conjugated antigen, harvesting B cells from the animal, creating a hybridoma form the harvested B cells, and screening the hybridomas for specificity for the native antigen. In a particular embodiment, the carrier molecule is HSP70. In another embodiment, the animal has an intact immune system. In yet another embodiment, the animal is a mammal, In further embodiments, B cells are harvested from ascites, spleen, lymph nodes, or blood, either individually or in combination. In yet further embodiments, the hybridoma is created using an immortal cell, such as, but not limited to a mouse myeloma cell, an immortal human cell, or an immortal rat cell. In other embodiments, the screening for specificity is done by a method chosen from the group consisting of radioimmunoassay, enzyme-linked immunosorbant assay, "sandwich" immunoassay, immunoradiometric assay, gel diffusion precipitation reaction, immunodiffusion assay, *in situ* immunoassay, western blot, precipitation reaction, agglutination assay, complement fixation assay, immunofluorescence assay, protein A assay, virus visualization assay, biological activity modulation asay, and immunoelectrophoresis assay.

The present invention further relates to methods of producing monoclonal antibodies specific to E7 oncoprotein comprising conjugating the E7 oncoprotein chemically to a carrier molecule wherein the carrier molecule is a heat-shock protein, immunizing an animal with the conjugated antigen, harvesting B cells from the animal, creating a hybridoma from the harvested B cells, and screening the hybridomas for specificity to the native E7 oncoprotein. In another embodiment, the chemical conjugation comprises creating a plasmid with an oligonucleotide sequence encoding E7 oncoprotein and an oligonucleotide sequence encoding HSP70; and transfecting a host cell with the plasmid, wherein the host cell transcribes the oligonucleotide sequences into the conjugated E7 oncoprotein. In yet another embodiment, the oligonucleotide sequence encoding E7 oncoprotein is SEQ ID NO: 1. In a further embodiment, the oligonucleotide sequence encoding E7 oncoprotein is SEQ ID NO: 3. In yet a further embodiment, the oligonucleotide sequence encoding HSP70 is SEQ ID NO: 5. In other embodiments, the host cell is *E. coli.* In an embodiment, the carrier molecule is HSP70. In another embodiment, the animal has an intact immune system. In yet another embodiment, the animal is a mammal. In further embodiments, B cells are harvested from ascites, spleen, lymph nodes, or blood, either individually or in combination. In yet further embodiments, the hybridoma is created using an immortal cell, such as, but not limited to a mouse myeloma cell, an immortal human cell, or an immortal rat cell. In a particular embodiment, the mouse myeloma cell is a Sp2/0-Ag14 mycloma cell. In other embodiments, the screening for specificity is done by a method chosen from the group consisting of radioimmunoassay, enzyme-linked immunosorbant assay, "sandwich" immunoassay, immunoradiometric assay, gel diffusion precipitation reaction, immunodiffusion assay, *in situ* immunoassay, western blot, precipitation reaction, agglutination assay, complement fixation assay, immunofluorescence assay, protein A assay, virus visualization assay, biological activity modulation asay, and immunoelectrophoresis assay.

The present invention further provides compositions comprising monoclonal antibodies specific to E7 oncoprotein produced by the method comprising conjugating the E7 oncoprotein chemically to a carrier molecule wherein the carrier molecule is a heat-shock protein, immunizing an animal with the conjugated antigen, harvesting B cells from the animal, creating a hybridoma from the harvested B cells, and screening the hybridomas for specificity to the native E7 oncoprotein. In another embodiment, the chemical conjugation comprises creating a plasmid with an oligonucleotide sequence encoding E7 oncoprotein and an oligonucleotide sequence encoding HSP70; and transfecting a host cell with the plasmid, wherein the host cell transcribes the oligonucleotide sequences into the conjugated E7 oncoprotein. In yet another embodiment, the oligonucleotide sequence encoding E7 oncoprotein is SEQ ID NO: 1. In a further embodiment, the oligonucleotide sequence encoding E7 oncoprotein is SEQ ID NO: 3. In yet a further embodiment, the oligonucleotide sequence encoding HSP70 is SEQ ID NO: 5. In other embodiments, the host cell is *E. coli.* In an embodiment, the carrier molecule is HSP70. In another embodiment, the animal has an intact immune system. In yet another embodiment, the animal is a mammal. In further embodiments, B bells are harvested from ascites, spleen, lymph nodes, or blood, either individually or in combination. In yet further embodiments, the hybridoma is created using an immortal cell, such as, but not limited to a mouse myeloma cell, an immortal human cell, or an immortal rat cell. In a particular embodiment, the mouse myeloma cell is an Sp2/0-Ag14 myeloma cell. In other embodiments, the screening for specificity is done by a method chosen from the group consisting of radioimmunoassay, enzyme-linked immunosorbant assay, "sandwich" immunoassay, immunoradiometric assay, gel diffusion precipitation reaction, immunodiffusion assay, *in situ* immunoassay, western blot, precipitation reaction, agglutination assay, complement fixation assay, immunofluorescence assay, protein A assay, virus visualization assay, biological activity modulation asay, and immunoelectrophoresis assay.

The present invention also provides for methods of using monoclonal antibodies specific to E7 oncoprotein for the detection of cervical intraepithelial neoplasia comprising obtaining a specimen of cervical epithelial cells and screening the specimen for the presence of E7 oncoprotein. In particular embodiments, the screening method for the presence of E7 oncoprotein is chosen from the group consisting of radioimmunoassay, enzyme-linked immunosorbant assay, "sandwich" immunoassay, immunoradiometric assay, gel diffusion precipitation reaction, immunodiffusion assay, *in situ* immunoassay, western blot, precipitation reaction, agglutination assay, complement fixation assay, immunofluorescence assay, protein A assay, virus visualization assay, biological activity modulation asay, and immunoelectrophoresis assay. In another embodiment, the presence of E7 oncoprotein is equal to or greater than 0.05ng/ml. In yet another embodiments, the monoclonal antibodies comprise of at least two immunoglobulin isotypes. In further embodiments, one immunoglobulin isotype is IgG2a, and another is IgG2b. In yet another embodiment, one immunoglobulin isotype has specificity for a different antigenic determinant than the second immunoglobulin isotype.

The present invention further provides kits for determing if a subject is at risk for developing cervical intraepithelial neoplasi comprising at least one reagent that specifically detects E7 oncoprotein, and instructions for determining that the subject is at increased risk of developing cervical intraepithelial neoplasia. In an embodiment, the regeant is monoclonal antibodies specific to E7 oncoprotein.

The present invention provides as well methods of producing monoclonal antibodies specific to a Prion protein comprising conjugating the Priori protein chemically to a carrier molecule wherein the carrier is a heat-shock protein, immunizing an animal with the conjugated antigen, harvesting B cells from the animal, creating a hybridoma from the harvested B cells, and screening the hybridomas for specificity to the native Prion protein. In particular embodiments, the conjugating is performed chemically using glutaraldehyde. In other embodiments, the carrier molecule is HSP70. In another embodiment, the animal is a mouse. In a further embodiment, the screening is done using an enzyme-linked immunosorbent assay.

The present invention further provides kits for determining if a subject is at risk for developing spongiform encephalopathy comprising at least one reagent that specifically detects Prion protein and instructions for determing that subject is at increased risk of developing spongiform encephalopathy.

The present invention also includes methods of producing monoclonal antibodies specific to hyaluronic acid comprising conjugating hyaluronic acid chemically to a carrier molecule wherein the carrier protein is a heat-shock protein, immunizing an animal with the conjugated antigen, harvesting B cells from the animal, creating a hybridoma from the harvested B cells, and screening the hybridomas for specificity to the native hyaluronic acid.

The present invention further includes methods of producing monoclonal antibodies specific to matrix metalloprotease 3 comprising conjugating matrix metalloprotease 3 to a carrier molecule wherein the carrier protein is a heat-shock protein, immunizing an animal with the conjugated antigen, harvesting B cells from the animal, creating a hybridoma from the harvested B cells, and screening the hybridomas for specificity to the native matrix metalloprotease 3. In a particular embodiment, the conjugating is performed chemically using glutaraldehyde. In another embodiment, the carrier molecule is HSP70. In yet another embodiment, the animal is a mouse. In yet another embodiment, the screening is done using an enzyme-linked immunosorbent assay.

### Description of figures

Figure 1 shows how HSP70 facilitates the presentation of antigens.
Figure 2 shows a flow chart showing the pathology of Human Papilloma Virus. CIN refers to cervical intraepithelial neoplasia. HSV refers to herpes simplex virus. HLA refers to human leukocyte antigen.
Figure 3 shows the sequences of the primers for E7 gene amplification using PCR (SEQ ID NOS: 14-17).
Figure 4 shows the oligonucleotide (SEQ ID NO: 1) and amino acid (SEQ ID NO: 2) sequences for clone E7 oncoprotein from HPV16 with EcoRI and BamHI restriction sites.
Figure 5 shows the oligonucleotide (SEQ ID NO: 3) and amino acid (SEQ ID NO: 4) sequences for clone E7 oncoprotein from HPV18 with EcoRI and BamHI restriction sites.
Figure 6 shows a construct of the position where the HPV16 E7 and alternatively where the HPV18 E7 gene was inserted at the EcoRI-BamHI site in a pBluescipt SK (+) (Stratagene) plasmid (SEQ ID NO: 18).
Figure 7 shows a picture of an electrophoresis gel demonstrating isolated bands of E7 oncoprotein from *E. coli* lysates transfected with HPVI6 E7 and HPVI8 E7 genes.
Figure 8 shows the DNA templates (SEQ ID NOs.: 19-22) used for PCR amplification of DNA containing E7 genes. The templates are based on plasmid DNAs pHE716 and pHE718, which contain the HPV E7 genes.
Figure 9 shows a schematic diagram of the structure of the plasmid PQE30-E716-dnaK.
Figure 10 shows the oligonucleotide sequence for Plasmid pQE30-dnaK (SEQ ID NO: 5).
Figure 11 shows a calibration curve for the quantitative determination of HPV 16 E7 oncoprotein under optimum conditions.

### Detailed description of the invention

### Definitions:

To facilitate understanding of the invention, a number of terms are defined below.

As used herein, the term "chemically conjugated," or "conjugating chemically" refers to linking the antigen to the carrier molecule. This linking can occur on the genetic level using recombinant technology, wherein a hybrid protein may be produced containing the amino acid sequences, or portions thereof, of both the antigen and the carrier molecule. This hybrid protein is produced by an oligonucleotide sequence encoding both the antigen and the carrier molecule, or portions thereof. This linking also includes covalent bonds created between the antigen and the carrier protein using other chemical reactions, such as, but not limited to glutaraldehyde reactions. Covalent bonds may also be created using a third molecule bridging the antigen to the carrier molecule. These cross-linkers are able to react with groups, such as but not limited to, primary amines, sulfhydryls, carbonyls, carbohydrates or carboxylic acids, on the antigen and the carrier molecule. Chemical conjugation also includes non-covalent linkage between the antigen and the carrier molecule.

As used herein, the term "carrier molecule" refers to any molecule that is chemically conjugated to the antigen of interest that enables an immune response resulting in antibodies specific to the native antigen.

As used herein, the terms "native," "natural" "native antigen," or "natural antigen" refers to the antigen as it occurs in nature. With respect to the invention, the "native antigens" are of "low immunogenicity." "Low immunogenicity" refers to the inability of the natural molecule to elicit a strong immune response resulting in the production of high affinity antibodies. The term "antigen", "antigen of interest," or specific molecules, such as-but not limited to E7 oncoprotein, Prion protein, matrix metalloprotease 3, hyaluronic acid-include the whole molecule or any portions thereof that maintain antigenic distinctiveness specific for the native antigen.

As used herein, the term "intact immune system" refers to an animal with a functional immune system capable of raising an immune response to a foreign antigen. The immune response includes the ability to generate B cells that secrete antibodies.

As used herein "amino acid sequence" refers to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

DNA molecules are said to have "5' ends" and "3' ends" because mononucleotides are reacted to make oligonucleotides or polynucleotides in a manner such that the 5' phosphate of one mononucleotide pentose ring is attached to the 3' oxygen of its neighbor in one direction via a phosphodiester linkage. Therefore, an end of an oligonucleotides or polynucleotide, referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide or polynucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements that direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, the terms "an oligonucleotide having a nucleotide sequence encoding" and "polynucleotide having a nucleotide sequence encoding," means a nucleic acid sequence comprising the coding region of a gene or, in other words, the nucleic acid sequence that encodes a gene product. The coding region may be present in a cDNA, genomic DNA, or RNA form. When present in a DNA form, the oligonucleotide or polynucleotide may be single-stranded (*i.e.,* the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. or a combination of both endogenous and exogenous control elements.

As used herein, the term "regulatory element" refers to a genetic element that controls some aspect of the expression of nucleic acid sequences. For example, a promoter is a regulatory element that facilitates the initiation of transcription of an operably linked coding region. Other regulatory elements include splicing signals, polyadenylation signals, termination signals, etc.

"Amplification" is a special case of nucleic acid replication involving template specificity. It is to be contrasted with non-specific template replication (*i.e.*, replication that is template-dependent but not dependent on a specific template). Template specificity is here distinguished from fidelity of replication (*i.e.*, synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Q replicase, MDV-1 RNA is the specific template for the replicase [D.L. Kacian et al., Proc. Natl. Acad Sci. USA, 69:3038 (1972)]. Other nucleic acids will not be replicated by this amplification enzyme. Similarly, in the case of T7 RNA polymerase, this amplification enzyme has a stringent specificity for its own promoters [Chamberlin et al., Nature, 228:227 (1970)]. In the case of T4 DNA ligase, the enzyme will not ligate the two oligonucleotides or polynucleotides, where there is a mismatch between the oligonucleotide or polynucleotide substrate and the template at the ligation junction [D.Y. Wu and R. B. Wallace, Genomics, 4:560 (1989)]. Finally, Taq and Pfu polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences [H.A. Erlich (ed.), PCR Technology, Stockton Press (1989)].

As used herein, the term "amplifiable nucleic acid" is used in reference to nucleic acids that may be amplified by any amplification method. It is contemplated that "amplifiable nucleic acid" will usually comprise "sample template."

As used herein, the term "sample template" refers to nucleic acid originating from a sample that is analyzed for the presence of "target" (defined below). In contrast, "background template" is used in reference to nucleic acid other than sample template that may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e.*, a sequence of nucleotides), whether occurring naturally as in a purified restriction digest or produced synthetically, recombinantly or by PCR amplification, that is capable of hybridizing to another oligonucleotide of interest. A probe may be single-stranded or double-stranded. Probes are useful in the detection, identification and isolation of particular gene sequences. It is contemplated that any probe used in the present invention will be labelled with any "reporter molecule," so that is detectable in any detection system, including, but not limited to enzyme (*e.g.*, ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the method of K.B. Mullis U.S. Patent Nos. 4,683,195, 4,683,202, and 4,965,188, hereby incorporated by reference, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (*i.e.,* denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified."

With PCR, it is possible to amplify a single copy of a specific target sequence in genomic DNA to a level detectable by several different methodologies (*e.g.*, hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "amplification reagents" refers to those reagents (deoxyribonucleotide triphosphates, buffer, etc.), needed for amplification except for primers, nucleic acid template, and the amplification enzyme. Typically, amplification reagents along with other reaction components are placed and contained in a reaction vessel (test tube, microwell, etc.).

As used herein, the terms "restriction endonucleases" and "restriction enzymes" refer to bacterial enzymes, each of which cut double-stranded DNA at or near a specific nucleotide sequence.

As used herein, the term "recombinant DNA molecule" as used herein refers to a DNA molecule that is comprised of segments of DNA joined together by means of molecular biological techniques.

The term "western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabelled antibodies.

The term "antigenic determinant" as used herein refers to that portion of an antigen that makes contact with a particular antibody (*i.e.*, an epitope). When a protein or fragment of a protein, or chemical moiety is used to immunize a host animal, numerous regions of the antigen may induce the production of antibodies that bind specifically to a given region or three-dimensional structure on the protein; these regions or structures are referred to as antigenic determinants. An antigenic determinant may compete with the intact antigen (*i.e.*, the "immunogen" used to elicit the immune response) for binding to an antibody.

As used herein, the term "vector" is used in reference to nucleic acid molecules that transfer DNA segment(s) from one cell to another. The term "vehicle" is sometimes used interchangeably with "vector."

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

As used herein, the term host cell refers to any eukaryotic or prokaryotic cell (*e.g.,* bacterial cells such as *E. coli,* yeast cells, mammalian cells, avian cells, amphibian cells, plant cells, fish cells, and insect cells), whether located in vitro or in vivo. For example, host cells may be located in a transgenic animal.

The term "transfection" as used herein refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

### 1. Certain Antigens Have Low Immunogenicity

There are many low-molecular-weight compounds, which are not capable of independently eliciting an immune response because of their small size. In an embodiment, a hapten can be conjugated with a carrier protein (such as, but not limited to, bovine serum albumin, egg albumin, or keyhole limpet hemocyanin), and this conjugate is used for immunization. In such embodiments, a variety of problems arise. For example, the primary immune response may be directed at the carrier protein and not at the hapten molecule, and this must be considered when testing sera from immunized animals and hybridoma supernatants. The testing should reveal whether the antibodies are specific to the hapten or to the conjugate as a whole.

Another problem can include changes in the three-dimensional structure (conformation) of the hapten that can occur during conjugation with the carrier. This may result in the antibodies being specific to the hapten within the conjugate, but are incapable of recognizing the free hapten molecule. In such cases, different conjugation variants can be tried such that different functional groups of the hapten are exposed on the carrier surface.

In yet another embodiment, immunization with immune complexes can be used when the antigen of interest is either a conservative low-immunogenic protein or is a minor antigenic determinant that already has a very low percentage of all specific antibodies directed to it during the immune response. In the first instance, the antibody component of the immune complex may be monoclonal antibodies with an unsatisfactory affinity obtained earlier after typical immunization. In the second case, monoclonal antibodies against strong antigenic determinants are used. An example of the successful use of this approach is the production of antibodies against human erythropoietin, α and γ interferons, and immunoglobulins of closely related species (such as, but not limited to, rat monoclonal antibodies against mouse IgG). *See* Table 1.

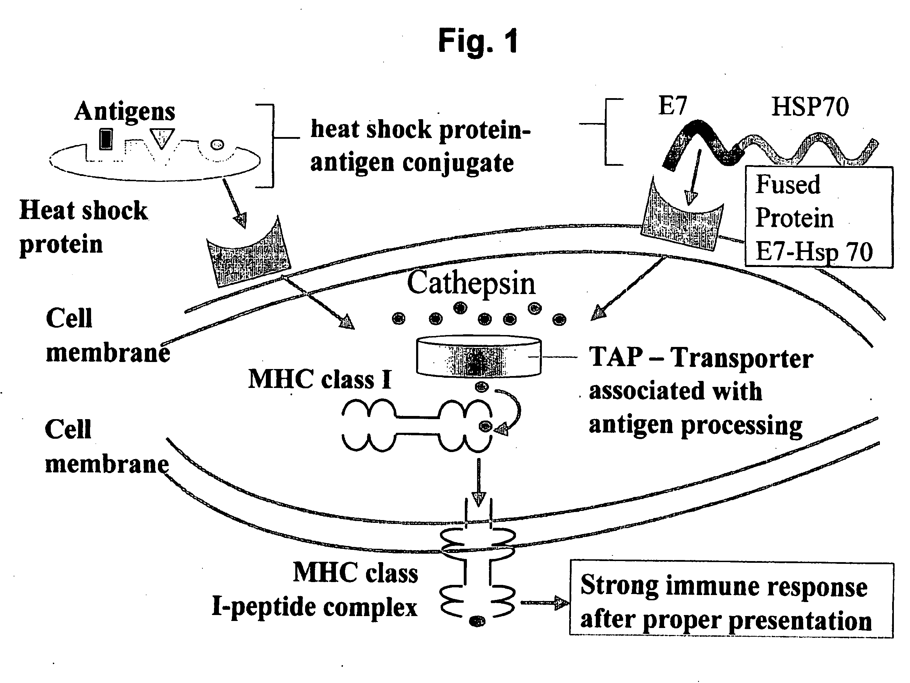

In other embodiments, some antigens have a very high degree of conservation and are incapable of eliciting any noticeable immune response, despite their protein nature and sufficient molecular weight (for example, but not limited to, hemoglobin, certain enzymes). In these particular embodiments, the antigens may be conjugated with the heat shock protein (such as, but not limited to, heat shock protein 70 kDa, otherwise known as HSP70). *See* Table 1, indicated as APP conjugate. The data shows that HSP70 facilitates the presentation of peptide fragments by exposing them in a certain way (*see* **Fig. 1**). Conjugation of proteins and peptides with HSP70 usually results in an abrupt intensification in the production of antibodies against these proteins and peptides. Yet, in other particular embodiments, the complete suppression of the immune response was seen with the conjugation of HSP70-interferon γ.

### II. Using HSP to Increase Immunogenicity

The methods described herein include a novel use of heat-shock protein as a vehicle to present the antigen of interest to produce antigen-specific antibodies for antigens that normally do not elicit an adequate immune response for the native molecule. Cells have evolved many mechanisms to help them survive in an unpredictable and hazardous world. One such mechanism is the heat-shock response, which is evoked by the exposure of cells to unusually high temperatures (such as, but not limited to, a fever), and other stresses including hypoxia, nutrient deprivation, oxygen radicals, metabolic disruption, viral infection, phagocytosis and transformation. This heat shock response includes the production of heat-shock proteins ("HSPs"). One such family of proteins is the HSP70 proteins. Members of the HSP70 protein family are found to function in the cytosol, mitochondria, as well as the endoplasmic reticulum of cells. HSP70 proteins each work with a small set of associated proteins when they help other proteins to fold. HSP70 and its associated proteins tend to bind to hydrophobic amino acids on a protein's surface, where they hydrolyze ATP, often binding and releasing their protein with each cycle of ATP hydrolysis. These repeated cycles help the target protein, for example, to refold.

Some of these heat-shock proteins help stabilize and repair partly denatured cell proteins. These HSPs also act as molecular chaperones. As chaperones, HSPs aid the conversion of a molten globule form of a protein to the correctly folded final compact conformation of the protein, protecting functional proteins from degradation, shuttling abnormal proteins to areas of degradation, transferring proteins between different intracellular compartments, assisting in the assembly of protein multimers, or aiding in the presentation of antigens with major histocompatibility complex (MHC) molecules on the surface of antigen-presenting cells.

It has been shown that the immune system responds vigorously to the heat-shock proteins of microbes, such as bacteria, parasites and fungi. The presence of foreign HSP triggers a prompt and potent immune response by the body. [Murray, P., Young, R., J. Bacteriol. 174:4193-4196 (1992)]. HSP70 is a major target of the immune response to many different pathogens including bacteria, fungi, helminth, and protozoan parasites. [Young, R. A., and Elliot, T. J., Cell, 59:5-8 (1989); Kaufmann, S.H., Immunol. Today, 11:129-136, (1990); Young, D.B., et al., Stress proteins and infectious diseases, p. 131-165 in Stress proteins in biology and medicine, Morimoto, R.I., et al., (eds.) Cold Spring Harbour Laboratory, Cold Spring Harbor, NY (1990); Young, R.A., Ann. Rev. Immunol., 8:401-420(1990)]. Immunization with a variety of pathogen HSPs induces a strong immune response and provides protection against diseases caused by these pathogens. [Suzue, K. and Young, R.A., J. Immunol., 156:873-876 (1996)].

The use of HSPs as vaccine vehicles based on the body's strong immune response began in the early 1990's. [Udono, H. and Srivastava, P.K., J. Exp. Med, 178:1391-1396, (1993); Udono, H., et al., Proc. Natl., Acad Sci. USA, 91:3077-3081, (1994); Suto, R. and Srivastava, P.K., Science, 269:1585-1588, (1995); Blachere, N.E., et al., J. Exp. Med., 186:1315-1322, (1997); Tamura, Y. P., et al., Science, 278:117-120, (1997); Nair, S., et al., J. Immunol., 162:6426-6432 (1999)]. The chemical conjugation [Lussow, A.R., et al., Eur. J. Immunol. 21:2297-2302 (1991); Barrios, C., et al., Eur. J. Immunol., 22:1365-1372, (1992); and Perraut, R., et al., Clin. Exp. Immunol., 93:382-386 (1993); Suzue, K. and Young, R.A., J. Immunol.I, 56:873-876, (1996); Suzue, K., et al., Proc. Natl. Acad. Sci. USA, 94:13146-13151, (1997); Rico, A.I., et al., .Infect. Immun., 66:347-352, (1998), hereby incorporated by reference] of antigens to HSP70 can create potent and customized immunogens that can elicit MHC class I-restricted, CD8+ cytotoxic T cell responses sufficient to mediate rejection of tumors expressing the fusion partner. This same cellular machinery is employed in the present invention in order to create antigen specific antibodies specific to native unconjugated antigen. *See* **Fig. 1**.

The prior art describes the use of HSP70 for the stimulation of an immune response whereby stimulating activity was assessed according to the degree of cellular and humoral immunity induction. There is, however, a significant difference between the induction of antibody genesis and monoclonal antibody production. The presence of antibodies in the serum does not always lead to the production of high affinity monoclonal antibodies that are able to recognize natural molecules. In a particular embodiment, in producing monoclonal antibodies to E7 oncoproteins, antibody genesis was observed after immunization of mice with the purified recombinant protein, however, upon testing none of the clones recognized natural E7 in cell line lysates containing the HPV genome. The immune system appears to recognize some determinants of the recombinant proteins that don't exist in natural proteins. In other words, the antibodies produced reacted with the antigen used for immunization, but did not react with the natural protein.

The present invention is not limited to any particular mechanism, however, it is believed that the three-dimensional protein structure of the recombinant proteins was different from that of the natural proteins. Therefore, non-natural antigenic determinants would be presented to the immune system. In overcoming this problem, the present invention uses a natural mechanism of antigen presentation (using hybrid proteins including the protein or antigen of interest conjugated with heat shock proteins) to present natural conformational determinants to the immune system. In particular embodiments, this is accomplished by using HSP70.

In these particular embodiments, an antigen of interest is conjugated to HSP70 or any portion of HSP70 that allows the antigen of interest to be immunogenic. Conjugation may be achieved chemically. [Lussow, A.R., et al., Eur. J. Immunol. 21:2297-2302 (1991); Barrios, C., et al., Eur. J. Immunol., 22:1365-1372, (1992); and Perraut, R., et al., Clin. Exp. Immunol., 93:382-386 (1993); Suzue, K. and Young, R.A., J. Immunol.I, 156:873-876, (1996); Suzue, K., et al., Proc. Natl. Acad Sci. USA, 94:13146-13151, (1997); Rico, A.I., et al., Infect. Immun., 66:347-352, (1998)]. An antigen may be any protein, peptide, nucleotide sequence, or chemical moiety. HSP70 can be from any source including but not limited to, mammalian, reptilian, amphibian, ichthyoidal, avian, insectoid, bacterial, fungal, helminthian, protozoan, viral, and plant.

Once the antigen of interest is conjugated to an HSP70 molecule, the conjugate is introduced to a foreign intact immune system able to process the conjugate and raise an immune response to the immunization with the conjugate. Any animal can be immunized and is not limited to mammals, but typical laboratory examples include, rat, mouse, hamster, gerbil, guinea pig, rabbit, dog, monkey, chicken, goat, sheep, swine, camel, cow, horse, and cat.

### III. Immunization

The effectiveness of immunization directly determines whether high affinity monoclonal antibodies can be produced. Various techniques are well known in the art and are used to increase the effectiveness of immunization, some of which are described herein.

### A. Selecting the Subject for Immunization

In particular embodiments, there are three typical types of hybridoma systems: mouse, rat, and human. The mouse hybridoma is the most widely used, and the human hybridoma the least used. The invention is in no way limited to these examples. Any animal with an intact immune system may be used as a B cell source for hybridization with a myeloma or any other immortal cell, including but not limited immortal human and immortal rat cells, to create a hybridoma.

The BALB/c mouse line is used most often for the immunization of mice; all mouse myeloma lines originate from this line. In the case of low-immunogenic antigens, mice of another line can be immunized. These mice may produce a stronger immune response to certain antigens in comparison with BALB/c. First generation offspring, obtained by crossing BALB/c and the line used for immunization, can be used to grow ascites for hybridoma production.

In other embodiments, rats are used, the literature emphatically recommends the LOU/C line, but totally satisfactory results were obtained with rats from the WISTAR line.

### B. Mouse Immunization Schedules

### I. Short immunization schedule

In particular embodiments, the antigen is administered twice with a 2-week interval into the hind footpads Balb/c mice. In yet further embodiments, the amount of the antigen typically ranges from 5 to 200 µg per mouse (but is not limited to these ranges) depending on its availability and immunogenicity. For toxic antigens, the dose may be reduced to as low as 0.1 µg. For the first administration of antigen (round of immunization), the antigen solution ranges typically from 50-200 µl in volume (but is not limited to this particular range so long as an immune response is achieved) and is combined with with an adjuvant such as complete Freund's adjuvant ("CFA"). CFA is commonly known in the art and is as mineral oil with a suspension of killed mycobacteria. Other adjuvants may also be used, such as, but not limited to, as mineral gels (*e.g.,* aluminum hydroxide), and surface active substances (*e.g.,* lysolecithin, pluronic polyols, polyanions, peptides, oil emulsions, keyhole limpet hemocyanins, dinitrophenol, and potentially human adjuvants such as BCG ("Bacille Calmette-Guerin") and *Corynebacterium parvum.*) Vigorous mixing of the antigen solution with CFA produces a finely dispersed emulsion for delaying and prolonging the entry of the antigen into tissue. The second round of immunization is carried out as the first, but with the use of incomplete Freund's adjuvant (IFA), which contains no mycobacteria.

In yet further embodiments, with antigens of low immunogenicity, it is useful to immunize a group of animals with different antigen doses and using various immunization schedules. In a particular embodiment, on the third or fourth day after the second round of immunization, blood samples are taken and analyzed for their content of antibodies against the specified antigen. Animals with the highest titer are usually selected for analysis. There are, however, exceptions such as protein toxins used as antigens. These types of antigens may produce a high specific antibody titer, but is also combined with a high percentage of B lymphocytes killed by the toxin. In these cases, animals that do not have the highest titer must be selected and/or attention must be focused on the viability of cells for fusion. On the fourth day after the second round of immunization, the animal is sacrificed and the cells of popliteal lymph nodes are fused with myeloma cells. Other lymph nodes from the animal may also be used.

### 2. Long immunization schedules

In another embodiment, where the short immunization schedule is not effective, various types of long schedules can be tried. In this case, the animals are usually immunized at an interval of 2-4 weeks either intraperitoneally or subcutaneously (alternatively, but less often, intravenously or orally). CFA is used for the first round of immunization and IFA for the next one.

On days 10-14 after each round of immunization (beginning with the second round), blood from the immunized animals is tested for the content of specific antibodies as well known in the art. If the antibody titer has reached the desired level, the last round of immunization with the antigen (called boosting) is given intravenously without adjuvants. In preferred embodiments, 1/10 of the original dose is given. Due to the boosting, clones producing antibodies with a high affinity for the antigen are stimulated selectively.

B cells that secrete antibody specific to the native antigen are then harvested and screened for activity by methods known to those skilled in the art and as described herein. [Harlow and Lane, *Antibodies: A Laboratory Manual,* Cold Spring Harbor Laboratory, Press, New York, (1988)]. Some of these screening techniques include, but are not limited to, radioimmunoassay, ELISA (enzyme-linked immunosorbant assay), "sandwich" immunoassay, immunoradiometric assay, gel diffusion precipitation reaction, immunodiffusion assay, *in situ* immunoassay (*e.g.,* using colloidal gold, enzyme or radioisotope labels, for example), western blot, precipitation reactions, agglutination assay (*e.g.,* gel agglutination assays, hemagglutination assays, etc.), complement fixation assay, immunofluorescence assay, protein A assay, virus visualization assay, biological activity modulation asay, and immunoelectrophoresis assay, etc.

Once a clone is identified with at least 1:1000 antibody titer, a hybridoma is created by methods known to those skilled in the art and as described herein. [Harlow and Lane, Antibodies: A Laboratory Manual, Cold Spring Harbor Laboratory Press, New York, (1988); Kohler and Milstein, Nature, 256:495-497 (1975); Kozbor et al., Immunol. Tod., 4:72 (1983); Cole et al., in Monoclonal Antibodies and Cancer Therapy, Alan R. Liss, Inc. pp.77-96 (1985); PCT/US90/02545. Cote et al., Proc. Natl. Acad Sci. USA, 80:2026-2030 (1983)]. These monoclonal antibodies may then be used for therapies for disease (such as, but not limited to, cancer, viral infections, etc.), immunodiagnostics, immunochemistry, immunohistology, immunocytology, immunoaffinity chromatography, and genomic and proteomic research.

Table 1 provides particular embodiments utilizing the methods of the invention to create high affinity monoclonal antibodies to proteins that traditionally have low immunogenicity. These embodiments are only some examples and are in no way limiting. The first column from the left identifies the antigen of interest. The second column identifies the immunogen that is used to immunize an animal with an intact immune system. In this column, APP refers to the methods described herein wherein the antigen of interest is chemically conjugated to HSP70. Also in this column, KLH conjugate means a conjugate with keyhole limpet hemocyanin rather than HSP70. The third column shows the number of clones produced from the immunization and the fourth column shows the number of clones which are able to bind to the native antigen of interest. The fifth column shows the quantitation method used to identify whether the monoclonal antibodies were specific to the antigen of interest. ELISA is used to identify enzyme linked immunosorbent assay. WB is used to identify western Blot. IF is used to identify immunofluorescence. IAC is used to identify immunoaffinity chromatography. The last column provides a brief explanation for why the antigens do not elicit a strong immune response without conjugation.

**TABLE 1**

| **Murine Monoclonal Antibodies Produced Using Adjuvant Polypeptide (APP) Technology** | | | | | |
|---|---|---|---|---|---|
| ***Target*** | ***Immunogen*** | ***Total number of clones produced*** | ***Number of clones which recognize intact target*** | ***Methods*** | ***Problem*** |
| 1. Matrix Metalloproteinase 3 (MMP3), human | Recombinant catalytic domain of MMP-3, 40-212, mol. Mass 19.369 Da-intact | 0 | 0 | ELISA | Conservative, low-immunogenic, improper folding |
| | Recombinant catalytic domain of MMP-3,40-212, mol. Mass 19.369 Da-APP conjugate | 4 | 1 | ELISA, WB | |
| 2. Troponin T, cardio specific, human (TT) | 16-mer peptide specific to Troponin T cardiac isoform 1 -KLH conjugate | 7 | 0 | ELISA, WB | Multiple isoforms, cross-reactivity |
| | 16-mer peptide specific to Troponin T cardiac isoform 1 - APP conjugate | 3 | 2 | ELISA, WB | |
| 3. Erythro-poetin, human (EPO) | Recombinant, full size human erythropoetin - intact | 0 | 0 | ELISA | Highly conservative, low immunogenic |
| | Recombinant, full size human erythropoetin - denatured, 6M urea | 2 | 1 | ELISA, WB | |
| | Recombinant, full size human erythropoetin - immune complex with Mab | 0 | 0 | ELISA | |
| | Recombinant, full size human erythropoetin - APP conjugate | 5 | 3 | ELISA, WB | |
| 4. Neuron Specific Enolase (γγ-subunit), human (NSE) | Natural NSE from human brain, tetramer, mol.mass 160kDa - intact | 9 | 1 | ELISA, WB, IF | Conservative, low immunogenic, inactivated by coating |
| | Natural NSE from human brain, tetramer, mol.mass 160kDa - denatured, 6M urea | 0 | 0 | ELISA | |
| | Natural NSE from human brain, tetramer, mol.mass 160kDa - immune complex with Mab | 0 | 0 | ELISA | |
| | Natural NSE from human brain, tetramer, mol.mass 160kDa - APP conjugate | 2 | 2 | ELISA, WB | |
| 5. Tissue Plasminogen Activator, human (TPA) | Recombinant full size human proTPA, mol. Mass 46 KDa - APP conjugate | 7 | 5 | ELISA, WB, IAC, Enzymatic activity inhibition | Avoid cross-reactivity with amino-terminal fragment, produce Mab which binds proTPA in 1M GnHCl |
| 6. Parathormone, human (PTH) | N-terminal 1-34. synthetic peptide - APP conjugate | 5 | 5 | ELISA | Low immunogenic, small size, expensive need for matched Mab pair. |
| 7. Parathormone, human (PTH) | Mid-molecule 28-48 synthetic peptide - APP conjugate | 2 | 1 | ELISA | Low immunogenic, small size, expensive need for matched Mab pair. |
| 8. Parathormone, human (PTH) | Mid-molecule 44-68 synthetic peptide - APP conjugate | 2 | 1 | ELISA | Low immunogenic, small size, expensive need for matched Mab pair. |
| 9. Hemoglobin, human (Hb) | Natural Hb from pooled human erythrocytes - intact | 0 | 0 | ELISA | Highly conservative, low immunogenic, need to distinguish glycosylated/ deglycosylated isoforms |
| | Natural Hb from pooled human erythrocytes - denatured, 6M urea | 1 | 1 (glycosylated only) | ELISA | |
| | Natural Hb from pooled human erythrocytes - APP conjugate | 3 | 3 (both) | ELISA | |
| 10. Ghre-lin, human | Full-size 1-28 synthetic peptide, octanoyl-group free - APP conjugate | 3 | 3 | ELISA | Small-size, low immunogenic, conservative |
| 11. Connexin 40, human | 16-mer peptide specific to human Connexin 40 - KLH conjugate | 2 | 0 | ELISA | Low abundance |
| | 16-mer peptide specific to human Connexin 40 - APP conjugate | 3 | 3 | ELISA, WB, IF | |
| 12. Interferon gamma, human | Recombinant, full-size human interferon gamma, Mol.mass 16.7 kDa - intact | 3 | 2 | ELISA | Low-immunogenic, need to produce neutralizing Mab |
| | Recombinant, full-size human interferon gamma, Mol.mass 16.7 kDa - APP conjugate | 4 | 3 | ELISA | |
| 13. Influenza A virus (HSN1), haemagglutinin | Tentative 16aa and 18 aa peptides type-specific to H5 haemagg-lutinin 16 AA- APP conjugate | 5 | 2 | ELISA, WB | Low abundance, cross-reactivity |
| | Tentative 16aa and 18 aa peptides type-specific to H5 haemagg-lutinin 18 AA- APP conjugate | 2 | 1 | ELISA, WB | |
| 14. Oncoprotein E7 from human Papilloma virus type 18 (E7HPV18) | Recombinant full-size E7HPV 18, mol. Mass 11995 Da - intact E7 | 0 | 0 | ELISA, WB | Immunosuppression, low-immunogenic, olygomerization, improper folding |
| | Recombinant full-size E7HPV 18, mol. Mass 11995 Da - denatured, 6M urea | 0 | 0 | ELISA, WB | |
| | Recombinant full-size E7HPV 18, mol. Mass 11995 Da - recombinant fused protein E7HPV 18-APP | 3 | 3 | ELISA, WB, IF | |
| 15.Prion Protein human (PrP) | 13-mer peptide specific to 130-142 aa of PrP-APP conjugate | 5 | 3 | ELISA, WB | Highly conservative, low-immunogenic |
| 16. Clostridium botulinum Neurotoxin A (BoNT/A) | Four peptides specific to H-chain of BoNT/A-APP conjugate | In progress | | ELISA, WB | Highly toxic, low immunogenic after inactivation by formaldehyde |
| 17. Hyaluronic acid (HUA) | HUA-APP conjugate | 3 | 3 | ELISA | Highly conservative, non-immunogenic |

### IV. Monoclonal Antibodies for the Detection Human Papilloma Virus/Cervical Cancer

In an embodiment of the invention, the inventive method was used to develop monoclonal antibodies specific to Human Papilloma Virus ("HPV") E6 and E7 oncoproteins. The development of specific monoclonal antibodies to these oncoproteins have been very difficult because natural E6 and E7 oncoproteins are hard to purify due to their low concentration, and recombinant proteins tend to fold incorrectly and aggregate. Additionally, E6 and E7 oncoproteins have immunosuppressive activity. The antibodies produced using recombinant E6 and E7 oncoproteins as an immunizing agent did not have high affinities for the native E6 and E7 proteins. Because of this, the novel method was employed to create the antibodies, which can be then used for many applications as described herein.

### A. Cervical Cancer Background

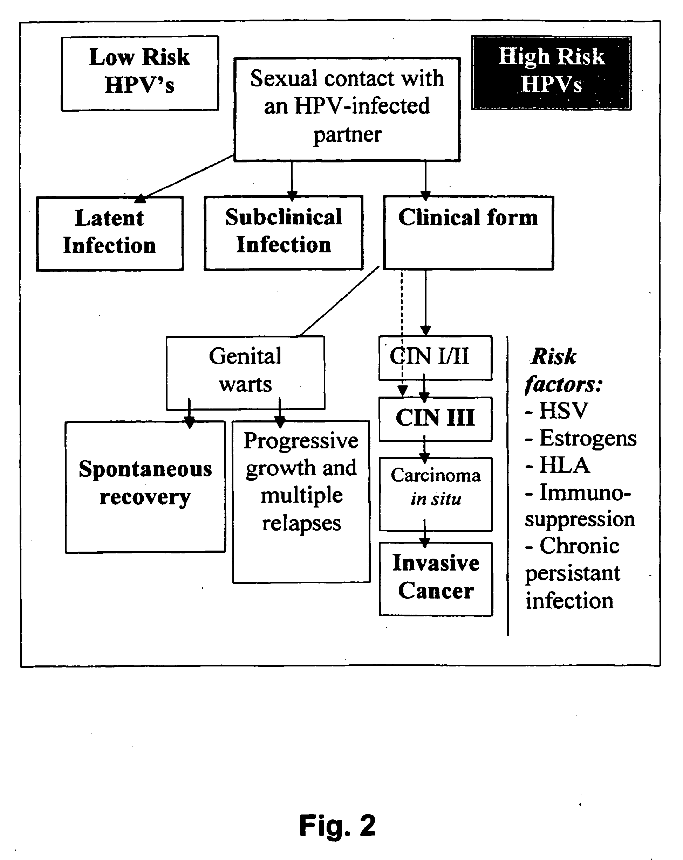

Cervical carcinoma is the second most prevalent cancer in women. A correlation has been shown between invasive cervical cancer and HPV based on epidemiological and virological studies (**Fig. 2**). A similar correlation has also been found between HPV and neoplasia of the external sex organs and anus, as well as invasive uterine carcinoma [Kiselev, V. I, et al., Interrelationship of Sexually Transmitted Viral Infections and Ontological Illnesses of the Urogenital Tract In Sexually Transmitted Diseases: Vaccines, Prevention, and Control. (ed.) D. I. Bernstein. Academic Press, London, Herald of Dermatology, No. 6:20-23 (2000)].

Cytological examinations (commonly known as Pap smears) still constitute the principal laboratory diagnostic technique to detect atypical multinucleated cells. This technique has been used for the early diagnosis of cervical cancer for a great many years. These procedures, however, do not detect more than 30% of all HPV carriers and cannot predict the risk of developing a more serious illness.

In studying the structure of HPV deoxyribonucleic acid ("DNA"), including the study of nine immunogenic proteins that are encoded by the DNA, together with the regularities of virus replication, immortalization, and its transformation of epithelial cells, a screening method was developed. The basis of which consisted of detecting biochemical, immunological, and genetic parameters that exhibit deviations from normal values when pathologies are present.

It is not always possible to interpret this type of data to make an early diagnosis, to assess the risk of developing cervical cancer, or to predict the effectiveness of treatment. This is because the measurement techniques used either do not have the requisite sensitivity and/or specificity, the parameters being measured do not directly reflect a cell degeneration process, or the measurements do not reflect it proportionally.

Techniques have been described for diagnosing cervical cancer by measuring antibody titers specific to HPV proteins in patient sera using enzyme immunoassays (which include, but are not limited to ELISA's) and immunoradiometric assays [*see,* European patents (EPs) No. 386734, 375555, 406542, and 523391]. This approach, however, has not lived up to expectations, since no reliable correlation has been found between an antibody titer and cervical cancer.

Techniques also exist for diagnosing and predicting the development of cervical cancer based on the typing and quantitative determination of the HPV DNA levels in a specimen undergoing study, using the polymerase chain reaction ("PCR"). [V. I. Kiselev, et al., The Polymerase Chain Reaction During the Diagnosis of Urogenital Infections: A Handbook for Physicians, Moscow, 2000; World Patent (WO) No. 00506645; and United States (U.S.) Patent No. 5,679,509].

The use of these techniques, however, have lead to considerable over diagnosis, since HPV infection is short-term in nature in roughly 80% of all cases, can culminate in spontaneous recovery, as well as elimination of the virus. *See* **Fig. 2**. Yet, a positive result for HPV DNA does, in most cases, reliably predict the development of cervical cancer.

The novel solution proposed by the present invention includes a method for the early diagnosis of cervical cancer and the determination of the risk of its development based on the quantitative immunological measurement of HPV E6 or E7 oncoproteins from a biopsic cell sample rather than patient sera [EP No. 1253924, A 61 K 38/18, 08/02/2001].

In a particular embodiment, the E6 and E7 proteins served as HPV markers and were detected in deparaffinized tissue samples via immunofluorescence technique, with digital computer technologies being used (for a quantitative determination) to analyze multidimensional visualized specimens. When the E6 or E7 protein content in biopsic samples exceeded the normal control values by more than 54%, the conclusion was reached that a heightened risk of development of cervical cancer existed. It has also been proposed that other biochemical markers can be used, such as, but not limited to the epidermal growth factor receptor and the insulin-like growth factor.

In yet another embodiment, a screening method was developed that was simpler and less costly that could easily lend itself to standardization due to a high level of sampling and analysis commonality. This screening method has a sensitivity that corresponds to the sensitivity of the immunoflourescence embodiment previously described. This screening method can be used for the early diagnosis of cervical cancer from cytological samples using the HPV E7 oncoprotein as a marker. The ability to use either diagnostic method depends upon the use of high affinity monoclonal antibodies specific to E6 or E7 oncoproteins. How these antibodies were produced are described herein.

### B. Development of Monoclonal Antibodies to E6 and E7

There is a liminal increase in the synthesis HPV E7 protein in reaction to the epithelial cell degeneration process that is preceded by viral DNA integration into cellular DNA. In a particular embodiment, an E7 gene was isolated from human specimens and cloned, then expressed in *Escherichia coli.* Recombinant E7 protein isolated from the bacterial cells was then used to immunize mice. In most cases, however, the antibodies produced by immunization with the recombinant proteins did not interact with the natural protein, since they recognize other determinants. In order to overcome this problem, the method described above was used, wherein the E7 protein was conjugated to HSP70 and then used as the antigen for immunization.

In particular embodiments, two types of highly sensitive monoclonal antibodies against an E7 protein of natural origin were produced, which cross-reacted with the two types of "high-risk" HPVs (HPV16 and HPV18). This cross-reactivity allows detection of 80-85% of all patients with HPV associated neoplasia. The paired use of these two types of monoclonal antibodies, which react to different E7 protein antigenic determinants, enhances the specificity and ensures the absence of a background in diagnostic screens.

### C. Screening Methods for HPV

In a particular embodiment, a method for the early and pre-clinical diagnosis of cervical cancer comprises of the quantitative immunological determination of a human papilloma virus (HPV) oncoprotein in a patient biopsic cell sample. The biopsic cell sample is lysed and the cell lysate supernatant is analyzed for HPV E7 protein using monoclonal antibody pairs. In a particular embodiment, the monoclonal antibody pairs belong to the IgG2a and IgG2b subisotypes and they recognize various antigenic determinants of the E7 protein. In a particular embodiment, the IgG2a 716-321, 716-325, 716-332, and 716-343 antibodies bind to at least one E7 antigenic determinant. In another embodiment the IgG2b 716-281 and 716-288 antibodies bind to at least one other E7 antigenic determinant. In another embodiment, one of the two antibody groups is conjugated to an enzyme label as known to those skilled in the art. [*See* Harlow and Lane, Antibodies: A Laboratory Manual, Cold Spring Harbor Laboratory Press, New York, (1988)]. These antibodies are then used in an enzyme linked immunosorbent assay ("ELISA") as well known to those skilled in the art. In a particular embodiment, wherein the E7 protein concentration in a sample is equal to or greater than 0.05 nanograms (ng) per milliliter (ml) a diagnosis of early stage cervical cancer or the risk of its development is positively made. In other particular embodiments, the quantitative analysis performance conditions were optimized by means of varying the concentration of antibodies for coating polystyrene plates, the conjugate dilution, the buffer solution composition, and the time and temperature of incubation for the ELISAs as well known in the art.

In a preferred embodiment, antibody 716-281 specific to one antigenic determinant of the E7 oncoprotein was used for the primary binding of the protein and antibody 715-332 specific to another E7 antigenic determinant was conjugated to an enzyme label. In yet another embodiment, the monoclonal antibodies used are specific to HPV16 E7, but also cross-react with HPV18 E7. HPV16 and HPV18 are the two most prevalent types of "high-risk" HPVs.

In a further embodiment, when a sample is positive for the E7 protein using the ELISA screening method, typing of the HPV is performed. The HPV type may be determined via various methods, including, but not limited to, using polymerase chain reaction, and *in situ* DNA hybridization.

### V. Monoclonal Antibodies Specific to Prion Protein.

In another embodiment of the invention, the inventive method was used to develop monoclonal antibodies against Prion protein ("PrP"). It is generally believed that a possible cause of spongiform encephalopathy (BSE, mad cow disease) is a conformational transition of a normal Prion protein into a pathogenic isoform. As the pathogenic isoform of PrP accumulates there is subsequent neuronal degradation. Monoclonal antibodies to this protein would be useful as a diagnostic tool for BSE. The main difficulty in producing monoclonal antibodies for this protein is a highly conserved structure of PrP. Multiple immunizations of Balb/c mice with a full-size recombinant bovine PrP both in native and denatured forms did not result in eliciting a sufficient immune response nor the generation of primary positive clones. In order to produce a monoclonal antibody to PrP, a conformational analysis of the PrP molecule was done and three amino acid sequences were selected. These three peptide sequences were from the N-terminal, mid-molecule and C-terminal parts of bovine PrP:

| | | |
|---|---|---|
| G5: | 25-36/62-69 | KKRPKPGGGWNT...QPHGGGWG (20 aa) (SEQ ID NOS: 6 and 7, respectively) |
| G3: | 109-121 | QWNKPSKPKTNIK (13aa) (SEQ ID NO: 8) |
| G4: | 226-242 | ITQYQRESQAYYQRGAS (17aa) (SEQ ID NO: 9) |

In particular embodiments, these three peptides were chemically conjugated to HSP70 and then used to immunize Balb/c mice using the methods disclosed herein. After completing the immunization schedule, popliteal lymph nodes (other lymph nodes, ascites, blood or spleen may be used as well) were harvested for clones producing antibodies specific to PrP. Hybridomas were made as well known in the art and as described herein, and hybridomas were screened for production of high affinity monoclonal antibodies. Monoclonal antibodies with high affinity were identified using immunoassays as well known in the art, such as, but not limited to ELISAs. These monoclonal antibodies may then be used for therapies for disease, immunodiagnostics, immunochemistry, immunohistology, immunocytology, immunoaffinity chromatography, and genomic and proteomic research.

### VI. Monoclonal Antibodies Specific to Hyaluronic Acid.

In another embodiment of the invention, the inventive method is used to create monoclonal antibodies to hyaluronic acid. Hyaluronic acid is a high molecular weight polymer composed of repeating dimeric units of glucuronic acid and N-acetylglucosamine which forms the core of the complex proteoglycan aggregates found in extracellular matrix. Hyaluronic acid plays a significant role in human connective tissue disorders such as, but not limited to, symptomatic osteoarthritis of the knee joint. Quantitative determination of hyaluronic acid and its degradation products is considered an important diagnostic parameter, which could be achieved with the use of monoclonal antibodies specific to hyaluronic acid.

Due to the non-protein nature of the polymer and its identity in many different species, ranging from bacteria and to mammals, the ability to create the high affinity antibodies of IgG1, IgG2a, IgG2b isotypes is very difficult.

In a particular embodiment, hyaluronic acid was conjugated with HSP70 for immunization and monoclonal antibody production as described for PrP. *See* Example 9. Monoclonal antibody screening was done using ELISA with the biotinylated hyaluronic acid and streptavidin-horse radish peroxidase as a secondary reagent for the color reaction. This type of ELISA is well known in the art. [Harlow and Lane, Antibodies: A Laboratory Manual, Cold Spring Harbor Laboratory Press, New York, (1988)].

As a result, three genetically stable hybridoma cell lines were produced, HU.D3 - IgG2a; HU.D9 - IgGI, HU.E3 - IgG3, each secreting high affinity monoclonal antibodies (IgG1 and IgG2a) against hyaluronic acid. These monoclonal antibodies may then be used for therapies for disease, immunodiagnostics, immunochemistry, immunohistology, immunocytology, immunoaffinity chromatography, and genomic and proteomic research.

### VII. Monoclonal Antibodies Specific to Matrix Metalloprotease 3

In another embodiment of the invention, the inventive method is used to create monoclonal antibodies to Matrix Metalloprotease 3 ("MMP3."). Proteins of the matrix metalloprotease (MMP) family are involved in the breakdown of extracellular matrix in normal physiological processes, such as embryonic development, reproduction, and tissue remodeling, as well as in disease processes, such as arthritis and metastasis. Most MMP's are secreted as inactive proproteins, which are activated when cleaved by extracellular proteases. MMP3 is an enzyme which degrades fibronectin, laminin, collagens III, IV, IX, and X, and cartilage proteoglycans. The enzyme is thought to be involved in wound repair, progression of atherosclerosis, and tumor initiation. MMP3 is reported to be implicated in rheumatoid arthritis and some forms of cancer. These monoclonal antibodies may then be used for therapies for disease, immunodiagnostics, immunochemistry, immunohistology, immunocytology, immunoaffinity chromatography, and genomic and proteomic research.

### VIII. Kits for Analyzing Risk of Disease Using Monoclonal Antibodies

The present invention also provides diagnostic kits. In some embodiments, the kits are useful for determining whether the subject is at risk of developing a disease or condition, for example, but not limited to, cervical cancer, spongiform encephalopathy, connective tissue disorders, arthritis and metastasis. The nature and use of a kit will depend on the specificity of the monoclonal antibody provided with the kit. The diagnostic kits are produced in a variety of ways. In some embodiments, the kits contain at least one monoclonal antibody that was generated using the methods of the invention for an antigen of traditionally low immunogenicity. In preferred embodiments, the kits contains reagents for conducting an ELISA or other similar immunoassay as well known in the art. In some embodiments, the kit contains instructions for determining whether the subject is at risk for developing the disease or condition. In preferred embodiments, the instructions specify that risk for developing the disease or condition is determined by detecting the presence or absence of the antigen of interest, such as, but not limited to E7 oncoprotein, PrP, MMP3, or hyaluronic acid. In some embodiments, the kits include ancillary reagents such as buffering agents, nucleic acid stabilizing reagents, protein stabilizing reagents, and signal producing systems (*e.g.*, florescence generating systems as Fret systems). The test kit may be packages in any suitable manner, typically with the elements in a single container or various containers as necessary along with a sheet of instructions for carrying out the test. In some embodiments, the kits also preferably include a positive control sample.

### EXAMPLES

### Example 1

The following examples are provided in order to demonstrate and further illustrate certain preferred embodiments and aspects of the present invention and are not to be construed as limiting the scope thereof.

In the experimental disclosure which follows, the following abbreviations apply: M (Molar); µM (micromolar); mM (millimolar); mol (moles); mmol (millimoles); µmol (micromoles); nmol (nanomoles); g (grams); mg (milligrams); µg (micrograms); ng (nanograms); 1 or L (liters); ml (milliliters); µl (microliters); cm (centimeters); mm (millimeters); µm (micrometers); nm (nanometers); °C (degrees Centigrade);); min. (minutes); sec. (seconds); % (percent); kb (kilobase); bp (base pair); PCR (polymerase chain reaction); aa (amino acid).

### Example 1

### Detection and Typing of Human Papilloma Virus (HPV)

### A. Collection of clinical material

Clinical specimens were prepared and transported as well known in the art. A sterile disposable probe was used to take a scrape of clinical specimens of cervical epithelial cells. The probe was washed with 500 µl of saline (0.83% NaCl) to remove the cells from the probe, with the solution collected into a 1.5ml covered Eppendorf tube. The specimen was then stored until use, usually at 4°C for up to five days and at -10 to -20°C for up to one month.

### B. Purification of DNA

DNA extraction methods are well known in the art and the methods to purify DNA for the present invention is not limited to this particular example. The specimen is thawed if frozen as well known in the art, and then centrifuged at 10, 000 rpm for I minute. A pellet is formed at the bottom and the supernatant is discarded. 100 µl of physiological solution is added to the tube and the pellet is homogenized. 500 µl of lysis solution is added and vortexed for 10 seconds. The specimen and tube are then incubated at 65°C. The lysis solution contains 6 M Guanidine thiocyanate (Sigma, USA), 10 mM EDTA (Sigma, USA), 1 % Triton X-100 (Sigma, USA), 10 mM Tris HCl, pH 7.3 (Sigma, USA), 1% 2-β-Mercaptoethanol (BioChemical, England). 20 µl of homogenizing Sorbent (Silica, Sigma, USA) is added and agitated for 10 minutes at 50-100 rpm. The specimen and tube are centrifuged at 10,000 rpm for I minute and the supernatant is discarded. 500 µl of wash solution is added and the tube was vortexed until the pellet has mixed into the solution and centrifuged again at 10,000 rpm for 1 minute, discarding again the supernatant. The wash solution contains 4 M Guanidine thiocyanate (Sigma, USA), 10 mM EDTA (Sigma, USA). I ml of 70% ethanol is added to the tube and vortexed well and centrifuged at 10,000 rpm for 1 minute discarding the supernatant. This ethanol wash is repeated. The tubes are then opened and the pellet allowed to dry for 5-10 minutes at 55°C. 100 µl of Nuclease Free Water (Promega, USA) is added to the tube and incubated for 5 minutes at 55°C. Specimens can then be used for polymerase chain reactions. Specimens can be stored at 4°C for up to a week or at -20°C for up to six months.

### C. Polymerase Chain Reactions ("PCR") to Type HPV

The PCR reactions were carried out in a 25 µl solution, containing 5 µl of template DNA, 67 mM Tris-HCl (pH 8.8), 16.6 mM (NH₄)₂SO₄, 1.5 mM MgCl₂, 100 mM dATP, dCTP, dTTP and dGTP, 10 pmol of each primer and 1 unit of Taq - polymerase (5 units/µl; Promega, USA). As well known in the art, a hot start technique was used. Thermal cycling conditions were: 94°C for 3 minutes, and then 40 cycles at 94°C for 30 seconds, 65°C for 30, seconds and 72°C for 30 seconds. The chart below shows the HPV type and primers used to amplify the DNA of those types of HPV.

| **Type HPV** | **Primer 5' - 3'** | **Amplicon Size (b.p.)** |
|---|---|---|
| HPV - 16 | E7-16-F *tga cag ctc aga gga gga g* (SEQ ID NO: 10) | 118 |
| | E7-16-R *gca caa ccg aag cgt aga g* (SEQ ID NO: 11) | |
| HPV - 18 | E7-18-F *gcg act cag agg aag aaa ac* (SEQ ID NO: 12) | 195 |
| | E7-18-R *ca aag gac agg gtg ttc aga* (SEQ ID NO: 13) | |

The amplification products were analyzed on 1.5% agarose gels and photographed under UV light (254 nm).

### Example 2

### Designing recombinant plasmids that encode the synthesis of E7 oncoproteins of the HPV6 and HPV18 types.

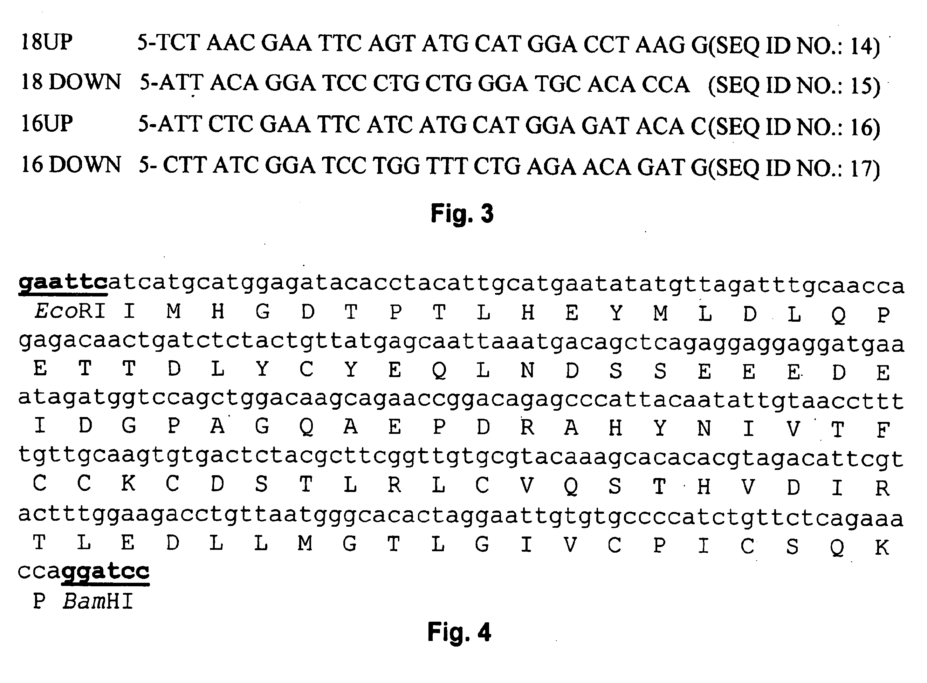

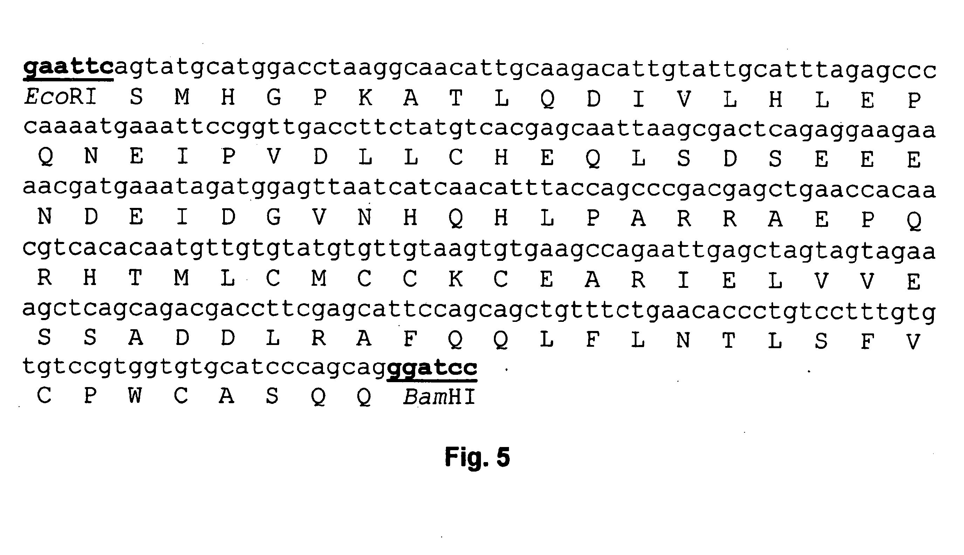

Biopsic tissue samples from the uterine cervices of patients diagnosed with cervical dysplasia served as the E7 gene source. The clinical specimens were examined for the presence of HPV and were typed using the polymerase chain reaction technique described herein. The E7 genes of the HPV16 and HPV18 types were amplified via the PCR technique using gene-specific primers [the 303-base-pair (bp) fragment for HPV16 and the 324-bp fragment for HPV 18] (SEQ ID NOS: 14-17), (*see* **Fig. 3**) whereupon these genes were cloned at the EcoRI-BamHI sites in a pBluescipt SK (+) (Stratagene) plasmid. The determination of the oligonucleotide sequences of the cloned genes demonstrated full agreement with GenBank data (for example, GenBank Accession No.: AFI25673 for HPV16 and AY262282 for HPV 18). (*See* **Figs. 4** **and** **5**, SEQ ID NOS: 1 and 3)

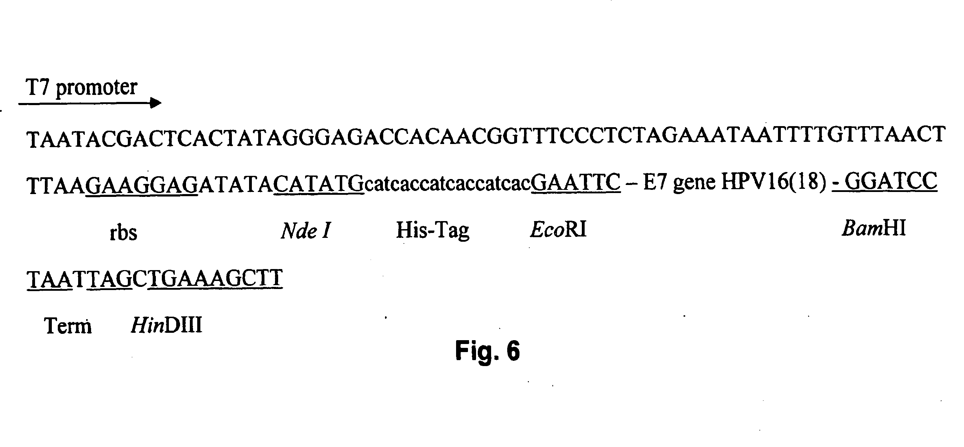

Next, translation termination sequences were incorporated into the genes near the 3' ends. The resultant genetic constructs, pHE716 and pHE718, are presented in **Fig. 6** for both HPV 16 and HPV 18 (SEQ ID NO: 18).

### Synthesis of E7 proteins of the HPV16 and HPV18 types in E. coli cells

The plasmid pHE716 encodes the protein E7-16 [Met(His)₆-GluPhelle-E716-GlySer [111 amino-acid residues ("aa"), 12.5 kilodaltons (kDa), and an isoelectric point (IEP) of 4.6]. And the plasmid pHE718 encodes the protein E7-18 [Met(His)₆-GluPheSer-E718-GlySer (117 aa, 13.5 kDa, and an IEP of 5.4]. It must be noted that when electrophoresis is performed in sodium dodecyl sulfate and a polyacrylamide gel ("SDS-PAGE") [Laemmli, U. K., Nature, 227:680-685 (1970)] this protein possesses an abnormal mobility (of approximately 21 kDa), which is in agreement with bibliographic data. [Jeon, J.-H., et al., Experimental and Molecular Medicine, 34:496-499,(2002)].

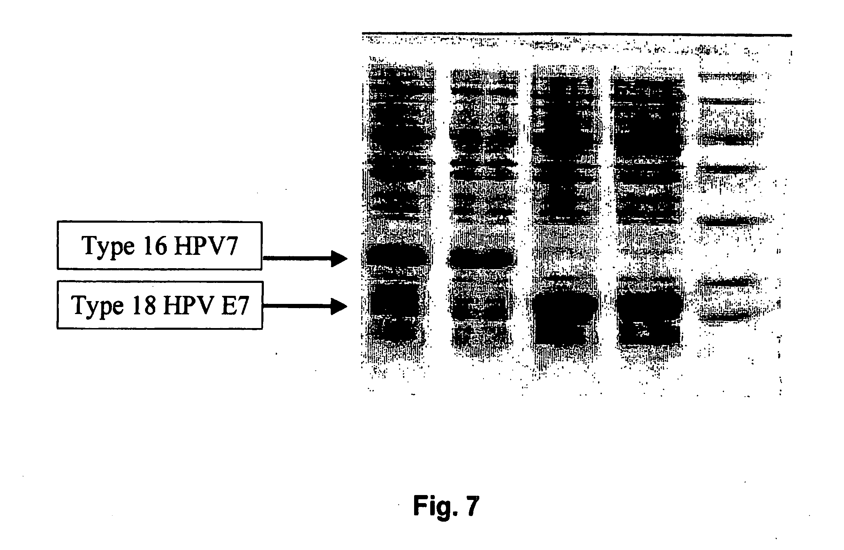

The BL21 (DE3) strain of *E. coli* (Stratagen, USA) was used for the expression of the resultant genes. Transfection was performed as well known in the art. Following ultrasonication, HPV16 E7 oncoprotein and HPV18 E7 oncoprotein were detected in a soluble cell protein fraction; therefore, chromatographic analysis on Ni-IIIA-agarose was performed under native conditions, without the addition of urea (**Fig. 7**).

More specifically, cells of *E. coli* strain BL21 (DE3) were transformed using the plasmids pHE716 and PHE718. The cells were incubated overnight at 37°C in Luria-Bertani ("LB") media with the addition of ampicillin (100 µg/ml) and glucose (0.2%). The cell were then diluted 1:25 with fresh LB media with ampicillin and incubated for 2-3 hours until cell density reached an optical density of OD₆₀₀=0.5. Optical density was measured on a Multiscan analyzer at a wavelength of 600 nm. Isopropyl β-D-thiogalactopyranoside ("IPTG") was then added to a concentration 0.2mM to the culture to a total volume of 50 ml and incubated for additional 3 hours. The cultures were then centrifuged at 5,000 rpm for 10 minutes, with the supernatant discarded. The pellet was resuspended in 1 ml of Tris-HCl 20 mM, pH 8, 0.3 M NaCl 0.1% Triton X-100 and sonicated for 15 seconds. The cultures were then centrifuged at 10,000 rpm for 10 minutes. The pellet was washed with Tris-HCL 20 mM, pH 8, 0.3 M NaCl 0.1% Triton X-100 (0.5 ml). The pellet is homogenized and resuspended in 5 ml of a buffer A (10mM Tris-HCl, pH 8, 6M urea, 0.4 M NaCl, 10mM β-Mercaptoethanol, 5mM Imidazole). The sample is then incubated at room temperature for 1 hour with agitation. The sample is then centrifuged at 10,000 rpm for 10 minutes. The pellet is discarded. A column with IDA-agarose (1 ml) was washed with a solution of NiSO4, then the column was equilibrated with buffer A. The supernatant was loaded onto the column with 5 ml of buffer A, and then 10 ml of buffer B (Buffer A with 10 mM imidazole). Elution was carried out with Buffer C (10 mM Tris-HCl, pH 8, 2M urea, 0.4 M NaCl, 10mM β-Mercaptoethanol, 0.3M imidazol) in 0.5 ml fractions. Ordinarily, the E7 protein from HPV16 and HPV18 usually eluted in fractions 1-3. The fractions containing the protein were pooled and dyalized overnight against 100 times the volume of the pooled fractions with a buffer containing 10mM Tris-HCl, pH 8, 150 mM NaCl.

### Example 3

### Expression of a hybrid protein consisting of E7 peptide of HPV type 16 (18) and DnaK (HSP70) M. tuberculosis.

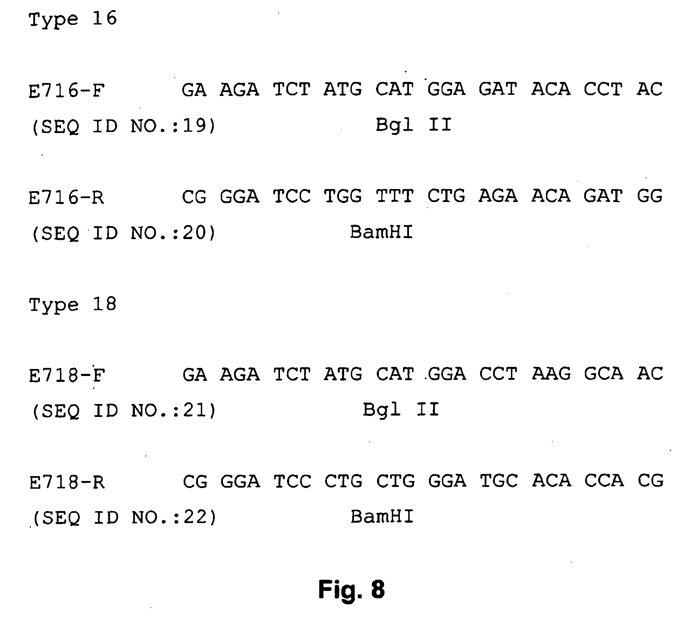

PCR was used to amplify the E7 gene of human papilloma virus, types 16 and 18. Plasmid DNAs pHE716 and pHE718 containing human papillomavirus (HPV) type 16 and 18 E7 genes were used as a template for PCR amplification of fragments containing E7 genes (SEQ ID NOS: 19-22). *See* **Fig. 8**. The initiation codon is in frame with the sequence encoding a 6HIS tag, and the stop codon is missing. DnaK protein was expressed using the cloned DNA fragments containing E7 genes transfected into a recombinant pQE30-based vector.

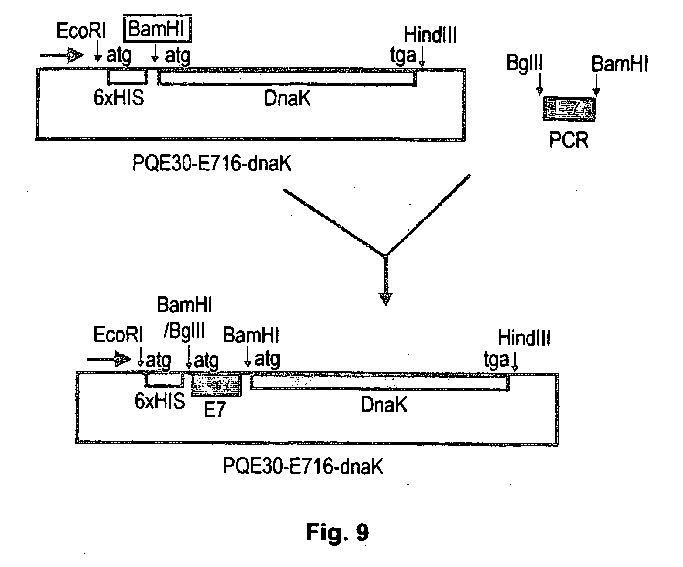

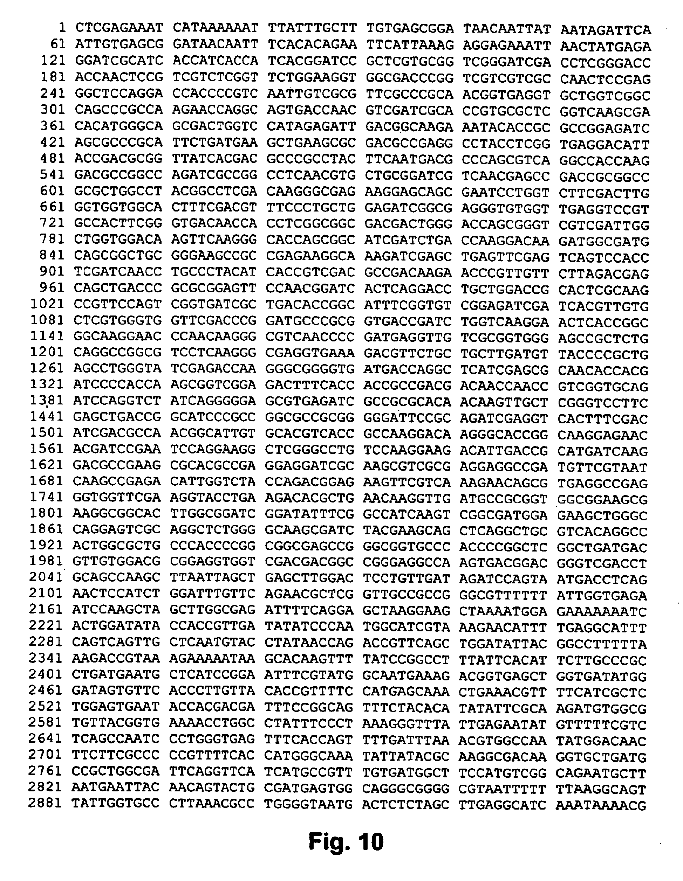

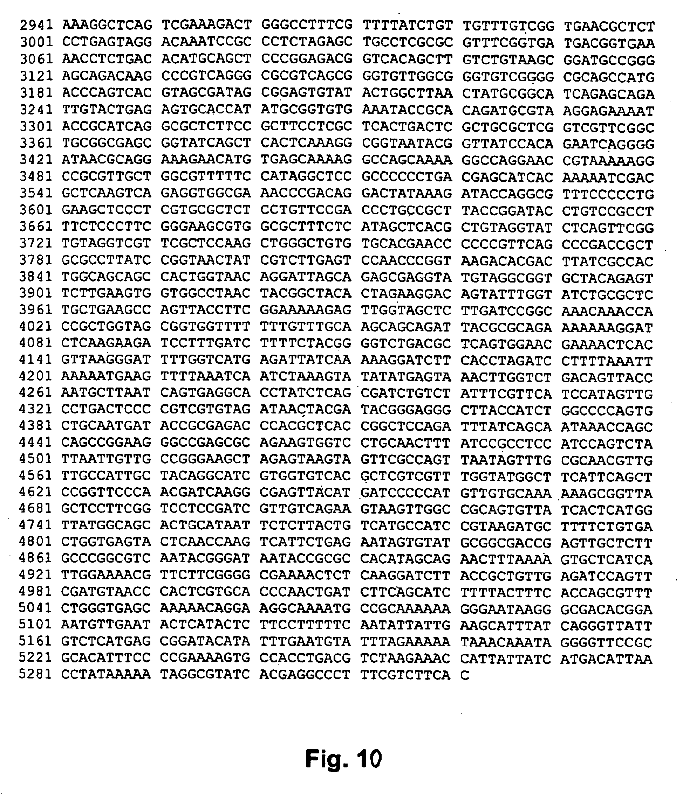

Recombinant vector pQE30-dnaK-Y was previously constructed and used as a basic plasmid, (**Fig. 9**) allowing expression of 6HIS-DnaK fusion protein. The nucleotide sequence of PQE30-dnaK (SEQ ID NO: 5) is shown in **Fig. 10**. To create this vector, PCR fragments containing E7 genes were digested with BamHI and BgIII and cloned into the BamHI site of pQE30-dnaK-Y. Recombinants carrying the inserts in correct orientation were identified using restriction analysis as well known in the art. Recombinant plasmids pQE30-E716-dnaK and pQE30-E718-dnaK allowed expression of hybrid proteins 6HIS-E7(type 16)-DnaK and 6HIS-E7(type 18)-DnaK, respectively.

This was accomplished by transfecting *E. coli* DLT1270 cells with pQE30-E716-dnaK and pQE30-E718-dnaK in separate cultures and incubating overnight. (DLT1270 cells were produced by integrating the *lac1* gene into the *E. coli* strain DH10B with the use of P1-trunsduction. DH 10B has the following genotype : 10 B ( ara D139 D (ara, leu) 7697 D (lac) X 74 galU galK rpsL deoR f80 lacZ DM15 end Al nupG recAl mcrA D (mrr hsdRMS mcr BC)). *See,* Grant, S., et al., Proc. Natl. Acad Sci. USA, 87:4645-4649 (1990).) The transfected DLT1270 cultures were grown in Luria-Bertani ("LB") broth that was diluted 100- fold and incubated at 37°C with shaking until a density measured by optical density reached an OD₆₀₀=0.5. Then isopropyl β-D-thiogalactopyranoside ("IPTG") was added up to 0.1 mM and the culture was grown as above for extra 2 hours. As well known in the art, IPTG induces the lacZ gene and is used to select cells transfected with plasmid of interest. Synthesis of the hybrid proteins of expected molecular weight was observed by SDS-PAGE. **Example 4**

### Isolation of Recombinant HSP70 Protein from E.Coli

### A. Buffer solutions

Buffer solutions are not prepared in advance. Buffer A is prepared before isolation procedure and used within one hour. Stock solution of 50 mM K2HPO4, pH 7 may be prepared in advance but must be used within three days. 10X PBS stock solution may also be prepared in advance. Storage of buffer solutions, and chromatographic procedures should be conducted at room temperature.

Buffer A comprises 25 mM N-(2-Hydroxyethyl)piperazine-N'-(2-ethanesulfonic acid) ("HEPES"), pH7.3, 0.1% Nonidet P-40 (w/v), 20% glycerol (v/v), 1 mM Phenylmethylsulphonyl fluoride ("PMSF"), and 0.5 M NaCl.

Buffer B comprises 50 mM K₂HPO₄, pH 7, 5 mM Imidazole, 0.5 M NaCl, pH is not adjusted.

Buffer C comprises 50 mM K₂HPO₄, pH 7, 150 mM Imidazole, 0.5 M NaCl.

Buffer D comprises 50 mM K₂HPO₄, pH 7, 50 mM EDTA, 0.5 M NaCl, pH is not adjusted.

PBS buffer comprises 10 mM K₂HPO₄, pH 7.5 and 0.145 M NaCl.

### B. Preparation of Chelating Column

Wash the I x 10 cm column with de-ionized water, forcing the bubbles from the lower filter, leaving a layer of water at the level of approximately 1 cm from the lower filter. Measure the necessary amount of Chelating Sepharose CL-6B (Amersham Pharmacia Biotech) to make a volume of 5ml. Using a pipette load the column with the Sepharose gel. Wash the column with 5 volumes of de-ionized water at the rate of 3 ml/minute. Then wash the column with 10 volumes of solution ZnCl₂ (concentration of 1 mg/ml (ICN)) at a rate of 3 ml/minute. Then wash the column with 10 volumes of de-ionized water at a rate of 3 ml/minute. Connect the column to a UV detector (280nm). Then wash the column with 10 volumes of water at a rate of 3 ml/ minute. The column is now prepared for isolation of protein from *E. coli* cells grown in 500 ml of culture medium.

### D. Preparation of E. coli

Procedures are to be conducted at 4°C as quickly as possible. First, wash the 1 gram *E. coli* (wet biomass) with 2 volumes of PBS 5 times by centrifuging at 5000 rpm for 15 seconds each cycle and then resuspending bacterial cells. Dilute cells after final wash in 5 ml PBS. The cells are then sonicated (20 kHz, 50-60 W) 6 times for 30 seconds. The resulting solution is centrifuged at 12,000 rpm for 10 minutes. The pellet is discarded, and the supernatant is loaded onto to the column.

### E. Separation of Protein

After the supernatant has been loaded, the column is washed with the column 10 volumes of Buffer A. This is followed by 5 volumes of Buffer B. Protein fractions are eluted with 5 ml Buffer C and collected.

### F. Dialysis of the protein recovered from the column

The pooled fractions are placed in a dialysis bag and is dialyzed for 30 minutes against 200 volumes of PBS at 0°C with constant stirring. After 30 minutes the solution is replaced with a fresh 200 volumes of PBS at 0°C also with constant stirring. The fractions are then dialyzed over night in 5 liters of PBS at 40°C. Determine the protein concentration by Bradford or Lowry protein quantitation methods as well known in the art. [Harlow and Lane, Antibodies: A Laboratory Manual, Cold Spring Harbor Laboratory Press, New York, (1988)]. Freeze the solution at-700°C and lyophilize.

### Example 5

### Production of mouse monoclonal antibodies against recombinant HPV16 and HPV18 E7 proteins

Balb/c mice [10 females weighing 16-18 grams (g)] were immunized twice in the hind foot pads, with a two-week interval between immunizations, using highly purified HPV 16 E7 and HPV18 E7. The HPV16 E7 and HPV18 E7 preparations of recombinant *E. coli* lysates were purified using single-stage metal-chelate chromatography. The first immunization contained 20 µg of protein diluted in 20 µl of phosphate buffered saline ("PBS") mixed with an equal volume of Freund's complete adjuvant. The second immunization also contained 20 µg of protein diluted in 20 µl of PBS, but was mixed with an equal volume of Freund's incomplete adjuvant. On the fourth day after the second immunization, lymphocytes from the popliteal lymph nodes were hybridized to Sp2/0-Ag14 myeloma cells (American Type Culture Collection ("ATCC") CRL 1581; ATCC CRL 8287; European Collection of Cell Cultures 85072401; DSMZ Human and Animal Cell Cultures ACC 146) using polyethylene glycol (PEG) 4000. The hybridomas were incubated in hypoxanthine-aminopterin-thymidine ("HAT") medium two weeks at 37°C with 5% CO₂. Surviving selected hybridomas were screened using an indirect ELISA. The positive cultures were cloned twice using the limited dilution technique. [*See* Campbell, A. M., Monoclonal Antibody and Immunosensor Technology in Laboratory Techniques in Biochemistry and Molecular Biology, vol. 23 (1991)]. The ELISA was performed by absorbing the recombinant HPV16 and HPV18 E7 proteins onto polystyrene plates at a concentration of 2 µg/ml by incubating the plates for 1 hour at 37°C, the plates were then washed 3 times with PBS, 50 µl of the tissue culture supernatant was added to the plates and incubated for 1 hour at 37°C. The monoclonal antibodies that bound to the immobilized antigen were detected using goat anti-mouse immunoglobulin G (IgG) [H+L] conjugated to peroxidase for a period of I hour at 37°C. A tetramethylbenzidine ("TMB") solution that contained hydrogen peroxide served as the substrate. Optical density was measured at 450 nm. Two groups of hybridomas were obtained for the HPV16 E7 protein that produced 716-281 and 716-288 (IgG2b) antibodies, as well as 716-321, 716-325, 716-332, and 716-343 (IgG2a) antibodies, which interacted equally well with HPV16 and HPV18 E7 in an indirect ELISA (*see* Table 2).

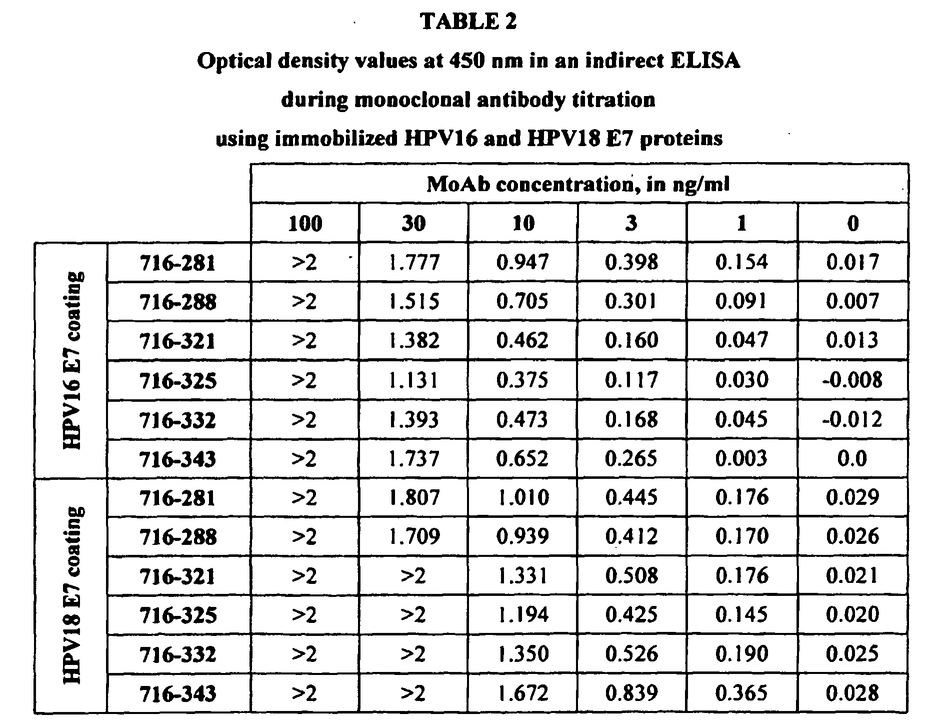

### Example 6

**Optimization of enzyme immunoassay performance conditions for the quantitative determination of HPV16 and HPV18 E7 proteins using monoclonal antibodies**

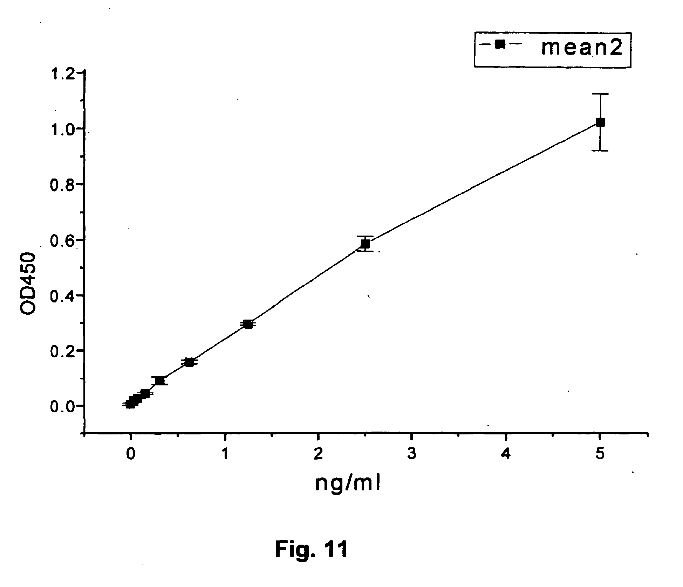

Monoclonal antibodies against HPV16 E7 were collected from mouse ascites and purified on a protein G-Sepharose affinity column (a purity of more than 95%). The antibodies were labeled with horseradish peroxidase using the periodate technique. All the paired monoclonal antibody combinations were checked for antigen affinity for HPV16 E7 by ELISA. To perform the monoclonal antibody pairing, one member of the monoclonal antibody pairs was immobilized on a polystyrene plate. Seven twofold dilutions of HPV16 E7 protein spanning a concentration range of 0.039-5.0 ng/ml were introduced to the monoclonal antibody immobilized on the tray. Immune complexes formed were detected by adding the second member of the monoclonal antibody pair conjugated to peroxidase with tetramethylbenzidine serving as the substrate. All of the monoclonal antibody pairs tested had the ability to form a ternary complex (a sandwich) - an immobilized antibody-E7-conjugated antibody- which suggests the oligomeric state of the HPV16 E7 protein in a solution, even at a concentration range of 10⁻¹²-10⁻⁹ moles (M). Despite the fact that all the monoclonal antibody combinations worked, pairs of identical antibodies, or antibodies that belonged to the same immunoglobulin isotype exhibited a lower level of sensitivity (the optical density value associated with a fixed HPV 16 E7 concentration and the calibration curve slope) than pairs of monoclonal antibody from different isotypes. In terms of sensitivity and the absence of a background, the best results were obtained when 716-281 monoclonal antibodies were bound to the plate and 716-332 monoclonal antibodies were used as the peroxidase conjugate. The assay using this monoclonal antibody pair was optimized by varying the concentration of the antibodies for absorption, the amount of conjugate dilution, the buffer solution composition, and the time and temperature for all stages of the ELISA. A calibration curve for the quantitative determination of HPV16 E7 under optimum conditions is presented in **Fig. 11**. The 716-288 monoclonal antibodies were absorbed from a 0.1 molar (M) carbonate buffer solution with a pH of 9.6 and a concentration of 5 µg/ml. The working dilution of the peroxidase-containing 716-332 MoAb conjugate was 1/5,000 in phosphate-buffered saline (PBS) that contained 0.2% bovine serum albumin (BSA) and 0.05% Tween 20. The substrate was tetramethylbenzidine (TMB). The protein concentration is shown along the x-axis, in ng/ml. Optical density at 45 nanometers (nm) is shown along the y-axis. The experimental error for three independent measurements is shown along this curve. As is apparent from the figure, the calibration curve is almost linear over an antigen concentration range of 0.039-5.0 ng/ml, and is also characterized by the total absence of a background. The presence of HPV16 E7 can be detected with as little as 40 picograms (pg) per ml, which is sufficient for determining the level of HPV16 E7 expression in transfected cells and clinical specimens (***see*** **Fig. 11**).

### Example 7

### Measurement of the E7 oncoprotein in cervical samples

A specimen from a uterine cervix scraping was placed into 1 ml of saline (0.83% NaCl) and frozen and thawed three times. The sample was then microcentrifuged (Eppendorf) for 10 minutes at 10,000 rpm. The supernatant was diluted 1:1 (v/v) in PBS-AT (PBS that contains 0.2% BSA and 0.1% Tween), whereupon 200 µl was introduced into the well of a plate previously bound by anti-HPV 16 E7 monoclonal antibodies at a concentration of 5µg/ml and was titrated with a two-fold serial dilution at 4 wells. Within this same plate, a purified recombinant HPV16 E7 protein was titrated from 4 ng/ml to 0.062 ng/ml as the standard. After one hour of incubation and washing, a peroxidase-labeled conjugated anti-HPV16 E7 monoclonal antibodies were introduced, incubation was continued for 1 hour, and a TMB reaction was developed. Optical density was measured on a Multiscan analyzer at a wavelength of 450 nm. A calibration curve was plotted based on the optical density (OD) values of the standard's dilutions, by means of which the E7 concentration in a sample was determined.

Screening studies were conducted (*n* = 100). Specimens from healthy patients among whom HPV was not detected, or among whom infection by "low-risk" HPV was detected, served as the control (*n* = 10).

The samples from all the healthy patients were negative for E7, or were slightly positive in certain cases. But, the E7 content was less than 0.05 ng/ml in all cases.

The positive samples exceeded the concentration threshold of 0.05 ng/ml. The data obtained are presented in Table 2.

**TABLE 2**

| **Determination of the E7 oncoprotein in cervical samples** | | | | | |
|---|---|---|---|---|---|
| Patient No. | Anti-E7 IgG | E7-16 | Total protein OD 1/20 | PCR confirmation | Diagnosis |
| P1 | **0.678** **0.418** | **0.1 ng/ml** | 0.084 | HPV 16 and 18 | CIN* III |
| P2 | **0.466** **0317** | **2.2 ng/ml** | 0.151 | HPV 31 | CIN I-II |
| P3 | **0.232** **0.127** | **0.8 ng/ml** | 0.152 | HPV with a high oncogenic risk | CIN II-III |
| P4 | **0.337** **0.158** | **3.5 ng/ml** | 0.228 | | CIN III and suspicion of cervical carcinoma |
| P5 | **0.268** | **10 ng/ml** | 0.041 | HPV 16 and 18 | CIN III and suspicion of cervical carcinoma |
| P6 | **0.422** **0.202** | **1 ng/ml** | 0.058 | HPV 16 and 18 | CIN II |
| P7 | **0.215** | **0.06 ng/ml** | 0.174 | HPV 16 and 18 | CIN II-III |
| P8 | **0.357** **0.164** | **03 ng/ml** | 0.089 | HPV 16 | CIN I-II |
| P9 | **0.412** **0.197** | **1.6 ng/ml** | 0.096 | HPV 18 | CIN II-III |
| P10 | **0.335** **0.130** | **0.9 ng/ml** | 0.165 | HPV 16 | CIN III and suspicion of cervical carcinoma |
| P11 | **0.280** | **1.2 ng/ml** | 0.180 | HPV 16 | CIN II |
| P12 | **0.554** **0.233** | **0.8 ng/ml** | 0.220 | HPV 16 | CIN II-III |
| P13 | **0.443** | **0.8 ng/ml** | 0.097 | HPV 16 | CIN I-II |
| P14 | **0.214** | **1.8 ng/ml** | 0.162 | HPV 16 | CIN II |

| | | | | | |
|---|---|---|---|---|---|
| *CIN-cervical intraepithelial neoplasia | | | | | |

As shown by the data, the results of the diagnostic screening method correspond with the medical diagnosis. The HPV typing data also correspond to the diagnostic data and can thereby be used to predict the risk of development of cervical cancer.

### Example 8

### Production of Monoclonal Antibodies to PrP.

Each peptide, G3, G4 and G5, was synthetically produced as known to those skilled in the art. [Fmoc Solid Phase Peptide Synthesis, A Practical Approach, eds. W.C. Chan and P.D. White, in The Practical Approach Series, series ed. B.D. Hames, Oxford University Press, (2000).] The peptides were lyophilized, and tested to be essentially salt-free, and 95% pure by HPLC. One hundred micrograms of each peptide was reconstituted with 0.1 ml 100 mM sodium phosphate buffer, pH 6.8 (coupling buffer) to create a "peptide solution". A solution of HSP70 (1mg/ml) was made with coupling buffer to make a "HSP70 solution". For each peptide, peptide solution (0.1 ml) was mixed with 0.2 ml HSP70 solution, containing 200 ug of the protein, yielding approximately 15-20 fold molar excess of the peptide over HSP70. Glutaraldehyde solution (25% glutaraldehyde with water) was added immediately up to 0.05% (v/v) to the mixture and incubated for 3 hours at room temperature with constant shaking. The coupling reaction was stopped by adding 0.5 M glycine solution to 0.1 M final concentration and incubated for another 30 minutes under the same conditions. The reaction mixture was then dialyzed against 100 ml PBS (10 mM Sodium Phosphate, 150 mM Sodium Chloride, pH 7.2) at 4°C overnight. The efficiency of the coupling reaction was checked by SDS-PAGE as well known in the art and results showed that there was no free HSP70 remaining. The conjugate produced was aliquoted and stored at -70°C without preservatives added.

Female Balb/c mice (18-20 g) were immunized twice into the hind footpads with 50 µl of the peptide-hsp70 conjugate solution with a two-week interval in between. At day 16 popliteal lymph nodes were harvested and processed for B cells for fusion with appropriate Balb/c myeloma cell line using conventional hybridoma technology as well known in the art and described herein.

The primary screening was done by indirect ELISA using G3, G4, G5 peptides, coated onto polystyrene plates with the following results:
Peptide G3 - hybridization yielded a small number of primary cultures, with even less number of positive ones, which later lost their activity.
Peptide G4 - 4 clones from different groups were established, thrice cloned.
Peptide G5 - 6 clones from different groups were established, thrice cloned.

Ascites were produced and also tested on corresponding peptides and full-size recombinant PrP. Table 3 shows the name of the clones isolated, the peptide each is specific to, and the immunoglobulin isotype. Table 4 shows the results of an indirect ELISA wherein the peptides used for making the conjugated protein is absorbed onto the polystyrene plate. Table 5 shows the results of an indirect ELISA wherein the native PrP protein is absorbed onto the polystyrene plate

**TABLE 3**

| **Immunogen and mouse Immunoglobulin sub-isotype specification of the Mab produced** | | |
|---|---|---|
| **Clone** | **Immunogen** | **Isotype** |
| 4G11 | G4-hsp70 | IgG2b |
| 4G29 | G4-hsp70 | IgG2b |
| 4G57 | G4-hsp70 | IgG2b |
| 4G69 | G4-hsp70 | IgG2a |
| 5G13 | G5-hsp70 | IgG3 |
| 5G29 | G5-hsp70 | IgG1 |
| 5G38 | G5-hsp70 | IgG3 |
| 5G48 | G5-hsp70 | IgG1 |
| 5G55 | G5-hsp70 | IgG1 |
| 5G77 | G5-hsp70 | IgG1 |

**TABLE4**

| **Monoclonal antibody indirect ELISA titration data (OD450 nm) using peptides for coating** | | | | | | | | | | |
|---|---|---|---|---|---|---|---|---|---|---|
| **Coating Antigen** | **Peptide G4** | | | | **Peptide G5** | | | | | |
| **Ascite Dilution** | **4G11** | **4G29** | **4G57** | **4G69** | **5G13** | **5G29** | **5G38** | **5G48** | **5G55** | **5G77** |
| **1/1000** | 1.428 | 1.527 | 1.479 | * | * | * | * | * | 1.604 | 1.643 |
| **1/3000** | 1.267 | 1.456 | 1.279 | * | 1.815 | * | * | 1.880 | 1.629 | 1.866 |
| **1/9000** | 1.016 | 1.412 | 1.094 | * | 1.824 | * | * | 1.838 | 1.572 | * |
| **1/27000** | 0.621 | 1.176 | 0.644 | * | 1.659 | 1.863 | 1.815 | 1.793 | 1.530 | 1.882 |
| **1/81000** | 0.284 | 0.877 | 0.273 | * | 1.306 | 1.691 | 1.018 | 1.613 | 1.421 | 1.811 |
| **1/243000** | 0.107 | 0.392 | 0.071 | 1.651 | 0.719 | 1.255 | 0.388 | 1.375 | 1.216 | 1.502 |
| **1/729000** | 0.035 | 0.118 | 0.010 | 0.755 | 0.247 | 0.725 | 0.114 | 0.752 | 0.873 | 0.961 |
| **1/2187000** | 0.015 | 0.043 | 0.003 | 0.270 | 0.044 | 0.330 | 0.039 | 0.294 | 0.481 | 0.537 |

**TABLE 5**

| **Monoclonal antibodies indirect ELISA titration data (OD450 nm) using PrP for coating** | | | | | | | | | | |
|---|---|---|---|---|---|---|---|---|---|---|
| **Coating Antigen** | **Prion protein** | | | | | | | | | |
| **Ascite Dilution** | **4G11** | **4G29** | **4G57** | **4G69** | **5G13** | **5G29** | **5G38** | **5G48** | **5G55** | **5G77** |
| **1/1000** | 0.139 | 0.089 | 0.137 | 0.257 | 0.193 | 0.296 | 0.138 | 0.129 | 1.832 | 0.159 |
| **1/3000** | 0.056 | 0.039 | 0.063 | 0.109 | 0.070 | 0.129 | 0.035 | 0.041 | 1.646 | 0.109 |
| **1/9000** | 0.036 | 0.031 | 0.044 | 0.070 | 0.030 | 0.093 | 0.014 | 0.018 | 1.726 | 0.088 |
| **1/27000** | 0.030 | 0.027 | 0.034 | 0.045 | 0.018 | 0.066 | 0.002 | 0.005 | 1.739 | 0.050 |
| **1/81000** | 0.027 | 0.025 | 0.030 | 0.027 | 0.005 | 0.033 | 0 | 0.004 | 1.742 | 0.032 |
| **1/243000** | 0.026 | 0.079 | 0.026 | 0.025 | 0 | 0.014 | 0 | 0.003 | 1.745 | 0.014 |
| **1/729000** | 0.025 | 0.023 | 0.025 | 0.026 | 0 | 0.009 | 0 | 0.001 | 1.550 | 0.003 |
| **1/2187000** | 0.031 | 0.024 | 0.024 | 0.024 | 0 | 0.001 | 0 | 0 | 1.168 | 0.002 |

All of the monoclonal antibodies shown above have high affinity for the immunogens used in generating them. In one particular embodiment, however, monoclonal antibody 5G55 strongly reacts with recombinant PrP. This antibody can be used for any applications utilizing ELISA, western blot and immunohistochemistry. The other remaining monoclonal antibodies show low to moderate binding specificity to recombinant PrP.

### Example 9

### Production of Monoclonal Antibodies to MMP 3.

As previously described for other antigens, BALB/c mice were immunized in their hind foot pads with MMP 3 (25µg/mouse). The mice received a second immunization 2 weeks later. On the fourth day after second immunization, lymph nodes were harvested and B cells from the nodes were fused with myeloma Sp2/0-Ag14 cells and placed into the 96-well plates. The immune sera was also collected and tested.

Table 6 shows the results of an indirect ELISA measured at OD 450 nm using MMP3 at 2 µg/ml in PBS that was absorbed onto polystyrene plates at 4°C overnight. All other incubations were performed in PBS with 0.5%BSA and Tween 20 as well known in the art. The results show that serum from mice immunized by MMP3 did not show any significant difference from serum of non-immune mice.

**TABLE 6**

| **Indirect ELISA measured at OD 450 nm using MMP3-Unconjugated** | |
|---|---|
| **Serum Dilution** | **OD₄₅₀ Meas.** |
| Immune serum | |
| 1:1000 | 0.52 |
| 1:3000 | 0.42 |
| 1:9000 | 0.36 |
| 1:27000 | 0.37 |
| Nonimmune serum | |
| 1:1000 | 0.53 |
| 1:3000 | 0.31 |
| 1:9000 | 0.25 |
| 1:27000 | 0.30 |

All primary clones were also tested by indirect ELISA on the MMP3 and by direct ELISA with biotin-labeled MMP3. No positive clones were created using unconjugated MMP3. MMP3 was then conjugated to HSP70 using the same method as described above for PrP using glutaraldehyde.

Conjugated MMP3 (50µg/mouse) was then used to immunize Balb/c mice as previously described. On the fourth day after the second immunization, lymph nodes were harvested and processed as well known in the art. Isolated B cells were fused with myeloma sp 2/0 cells and placed into the 96-well plates. The immune sera were also collected and tested. Results are shown on Table 7. Table 7 shows the results of an indirect ELISA measured at OD 450 nm using MMP3 2 µg/ml PBS that was absorbed onto polystyrene plates at 4°C overnight. All other incubations were performed in PBS with 0.5% bovine serum albumin ("BSA") and Tween 20 as well known in the art. The results show that serum from mice immunized with conjugated MMP3-HSP70 has significant affinity for MMP3 compared to serum of non-immune mice.

**TABLE 7**

| **Indirect ELISA measured at OD 450 nm using MMP3-Conjugated** | |
|---|---|
| **Serum Dilution** | **OD₄₅₀ Meas.** |
| Immune serum | |
| 1:1000 | 1.252 |
| 1:3000 | 0.835 |
| 1:9000 | 0.596 |
| 1:27000 | 0.452 |
| Nonimmune serum | |
| 1:1000 | 0.258 |
| 1:3000 | 0.268 |
| 1:9000 | 0.279 |
| 1:27000 | 0.277 |

All primary clones were also tested by indirect ELISA using MMP3 (2µg/ml) and direct ELISA with biotin-labeled MMP3 (500ng/ml). Eight positive clones were detected by indirect ELISA and 6 positive clones were detected by direct ELISA. All positive clones were cloned, four of them had stabile antibody production and were cloned 2-3 times. Table 8 identifies the clones and immunoglobulin isotypes. Table 9 shows the results of an indirect ELISA using 1 µg/ml of MMP3 absorbed onto the plate and direct ELISA using 0.5 µg/ml MMP3-biotin. The results show that the clones have a high affinity for MMP3.

**TABLE 8**

| **Monoclonal antibody producing clones for MMP3 and immunoglobulin isotypes** | |
|---|---|
| **Name of clone** | **Antibody isotypes** |
| MMP3H2 | IgG3 |
| MMP3H10 | IgG3 |
| MMP3G7 | IgG2a |
| MMP3F11 | G3 |

**TABLE 9**

| **Characteristics of the antibodies against MMP3** | | | | | | | | |
|---|---|---|---|---|---|---|---|---|
| MoAb, ng/ml | MMP3 G7, Indirect ELISA | MMP3 G7, Direct ELISA | MMP3 F11, Indirect ELISA | MMP3 F11, Direct ELISA | MMP3 H2, Indirect ELISA | MMP3 H2, Direct ELISA | MMP3 H10, Indirect ELISA | MMP3 H10 Direct ELISA |
| 10000 | **** | 0,093 | 0,772 | 0.474 | **** | 0,057 | **** | 0,102 |
| 3 000 | 1,765 | 0,020 | 0,295 | 0,472 | 1,824 | 0,090 | 1,809 | 0,055 |
| 1 000 | 1,795 | 0,023 | 0,182 | 0,384 | 1,782 | 0,126 | 1,829 | 0,077 |
| 300 | 1,830 | 0,037 | 0,044 | 0,269 | 1,607 | 0,088 | 1,793 | 0,025 |
| 100 | 1,745 | 0,009 | 0,021 | 0,147 | 1,248 | 0,038 | 1,683 | 0,017 |
| 30 | 1,470 | 0,000 | 0,015 | 0,069 | 0,719 | 0,033 | 1,391 | 0,019 |
| 10 | 0,957 | 0,000 | 0,000 | 0,057 | 0,295 | 0,003 | 0,941 | 0,004 |
| 3 | 0,498 | 0,000 | 0,000 | 0,051 | 0.099 | 0,000 | 0,463 | 0,005 |

Although the invention has been described in connection with specific preferred embodiments, it should be understood that the invention as claimed should not be unduly limited to such specific embodiments.

## Claims

1. A method of producing monoclonal antibodies specific to an antigen of low immunogenicity comprising:
a. conjugating the antigen chemically to a carrier molecule, wherein the carrier molecule is a heat-shock protein;
b. immunizing a non human animal with the conjugated antigen;
c. harvesting B cells from the animal;
d. creating a hybridoma from the harvested B cells; and
e. screening the hybridomas for specificity to the native antigen.

2. The method of claim 1, wherein the carrier molecule is HSP70.

3. The method of claim 1, wherein the animal has an intact immune system.

4. The method of claim 1, wherein the animal is a mammal.

5. The method of claim 1, wherein the B cells are harvested from ascites.

6. The method of claim 1, wherein the B cells are harvested from lymph nodes.

7. The method of claim 1, wherein the B cells are harvested from blood.

8. The method of claim 1, wherein the B cells are harvested from spleen.

9. The method of claim 1, wherein the hybridoma is created using an immortal mouse cell.

10. The method of claim 9, wherein the immortal mouse cell is a mouse myeloma cell.

11. The method of claim 1, wherein the hybridoma is created using an immortal human cell.

12. The method of claim 1, wherein the hybridoma is created using an immortal rat cell.

13. The method of claim 1, wherein the screening for specificity is done by a method chosen from the group consisting of radioimmunoassay, enzyme-linked immunosorbent assay, "sandwich" immunoassay, immunoradiometric assay, gel diffusion precipitation reaction, immunodiffusion assay, in situ immunoassay, western blot, precipitation reaction, agglutination assay, complement fixation assay, immunofluorescence assay, protein A assay, virus visualization assay, biological activity modulation assay, and immunoelectrophoresis assay.

14. The method of any of claims 1-13 wherein the antigen is E7 oncoprotein.

15. The method of claim 14, wherein the chemical conjugation comprises:
a. creating a plasmid with an nucleotide sequence encoding E7 oncoprotein and an nucleotide sequence encoding HSP70; and
b. transfecting a host cell with the plasmid, wherein the host cell transcribes the nucleotide sequences into the conjugated E7 oncoprotein.

16. The method of claim 15, wherein the nucleotide sequence encoding E7 oncoprotein is SEQ ID NO : 1.

17. The method of claim 15, wherein the nucleotide sequence encoding E7 oncoprotein is SEQ ID NO: 3.

18. The method of claim 15, wherein the nucleotide sequence encoding HSP70 is SEQ ID NO : 5.

19. The method of claim of claim 15, wherein the host cell is E. coli.

20. The method of claim 14, wherein the mouse myeloma cell is an Sp2/0-Agl 4 myeloma cell.

21. The method of any of claims 1-13 wherein the antigen is a Prion protein peptide.

22. The method of claim 21, wherein the conjugating is performed chemically using glutaraldehyde.

23. The method of claim 21, wherein the Prion protein peptide is SEQID NO: 6.

24. The method of claim 21, wherein the Prion protein peptide is SEQID NO: 7.

25. The method of claim 21, wherein the Prion protein peptide is SEQID NO: 9.

26. The method of any of claims 1-13 wherein the antigen is hyaluronic acid.

27. The method of any of claims 1-13 wherein the antigen is matrix metalloprotease 3.

28. The method of claim 27, wherein the conjugating is performed chemically using glutaraldehyde.

29. A composition produced by the method of any one of claims 16-18 and 23-25.

30. A method of using monoclonal antibodies specific to E7 oncoprotein made by the method of any one of claims 16-18 for the detection of cervical intraepithelial neoplasia comprising:
screening a specimen of cervical epithelial cells for the presence of E7 oncoprotein.

31. The method of claim 30, wherein the screening method for the presence of E7 oncoprotein is chosen from the group consisting of radioimmunoassay, enzyme-linked immunosorbent assay, "sandwich" immunoassay, immunoradiometric assay, gel diffusion precipitation reaction, immunodiffusion assay, in situ immunoassay, western blot, precipitation reaction, agglutination assay, complement fixation assay, immunofluorescence assay, protein A assay, virus visualization assay, biological activity modulation assay, and immunoelectrophoresis assay.

32. The method of claim 30, wherein the presence of E7 oncoprotein is equal to or greater than 0.05 ng/ml.

33. The method of claim 30, wherein the monoclonal antibodies comprise of at least two immunoglobulin isotypes.

34. The method of claim 33, wherein one immunoglobulin isotype is IgG2a.

35. The method of claim 33, wherein one immunoglobulin isotype is IgG2b.

36. The method of claim 33, wherein one immunoglobulin isotype has specificity for a different antigenic determinant than the second immunoglobulin isotype.

37. A kit for determining if a subject is at risk for developing cervical intraepithelial neoplasia comprising:
a. at least one reagent that specifically detects E7 oncoprotein wherein the reagent is the monoclonal antibody made by the method of any one of claims 16-18; and
b. instructions for determining that the subject is at increased risk of developing cervical intraepithelial neoplasia.

38. The kit of claim 37, wherein the reagent is the monoclonal antibodies of claim 29.

39. A kit for determining if a subject is at risk for developing spongiform encephalopathy comprising:
a. at least one reagent that specifically detects Prion protein wherein the reagent is the monoclonal antibodies made by the method of any one of claims 23-25; and
b. instructions for determining that the subject is at increased risk of developing spongiform encephalopathy.

## Patentansprüche

1. Verfahren zum Erzeugen monoklonaler Antikörper, die für ein Antigen mit geringer Immunogenität spezifisch sind, wobei das Verfahren folgendes umfaßt:
a. chemisches Konjugieren des Antigens mit einem Trägermolekül, wobei das Trägermolekül ein Hitzeschockprotein ist,
b. Immunisieren eines Tiers, welches kein Mensch ist, mit dem konjugierten Antigen,
c. Ernten von B-Zellen aus dem Tier,
d. Erzeugen eines Hybridoms aus den geernteten B-Zellen und
e. Screenen des Hybridoms hinsichtlich Spezifität für das native Antigen.

2. Verfahren nach Anspruch 1, wobei das Trägermolekül HSP70 ist.

3. Verfahren nach Anspruch 1, wobei das Tier ein intaktes Immunsystem hat.

4. Verfahren nach Anspruch 1, wobei das Tier ein Säuger ist.

5. Verfahren nach Anspruch 1, wobei die B-Zellen aus Aszites geerntet werden.

6. Verfahren nach Anspruch 1, wobei die B-Zellen aus Lymphknoten geerntet werden.

7. Verfahren nach Anspruch 1, wobei die B-Zellen aus Blut geerntet werden.

8. Verfahren nach Anspruch 1, wobei die B-Zellen aus der Milz geerntet werden.

9. Verfahren nach Anspruch 1, wobei das Hybridom unter Verwendung einer sich unbegrenzt vermehrenden Mauszelle erzeugt wird.

10. Verfahren nach Anspruch 9, wobei die sich unbegrenzt vermehrende Mauszelle eine Maus-Myelomzelle ist.

11. Verfahren nach Anspruch 1, wobei das Hybridom unter Verwendung einer sich unbegrenzt vermehrenden humanen Zelle erzeugt wird.

12. Verfahren nach Anspruch 1, wobei das Hybridom unter Verwendung einer sich unbegrenzt vermehrenden Rattenzelle erzeugt wird.

13. Verfahren nach Anspruch 1, wobei das Screenen hinsichtlich Spezifität unter Verwendung eines Verfahrens durchgeführt wird, das aus der Gruppe ausgewählt ist, bestehend aus Radioimmuntest, heterologem Enzym-Immuntest, "Sandwich"-Immuntest, immunradiometrischem Test, Geldiffusions-Präzipitations-Reaktion, Immundiffusionstest, *in situ*-Immuntest, Western Blot, Präzipitationsreaktion, Agglutinationstest, Komplementbindungstest, Immunfluoreszenztest, Protein A-Test, Virus-Visualisierungstest, Test auf Modulation der biologischen Aktivität und Immunelektroforesetest.

14. Verfahren nach einem der Ansprüche 1 bis 13, wobei das Antigen E7-Onkoprotein ist.

15. Verfahren nach Anspruch 14, wobei die chemische Konjugation folgendes umfaßt:
a. Erzeugen eines Plasmids mit einer Nukleotidsequenz, die E7-Onkoprotein codiert, und einer Nukleotidsequenz, die HSP70 codiert, und
b. Transfizieren einer Wirtszelle mit dem Plasmid, wobei die Wirtszelle die Nukleotidsequenzen in das konjugierte E7-Onkoprotein transkribiert.

16. Verfahren nach Anspruch 15, wobei die Nukleotidsequenz, die E7-Onkoprotein codiert, SEQ ID NO: 1 ist.

17. Verfahren nach Anspruch 15, wobei die Nukleotidsequenz, die E7-Onkoprotein codiert, SEQ ID NO: 3 ist.

18. Verfahren nach Anspruch 15, wobei die Nukleotidsequenz, die HSP70 codiert, SEQ ID NO: 5 ist.

19. Verfahren nach Anspruch 15, wobei die Wirtszelle E. coli ist.

20. Verfahren nach Anspruch 14, wobei die Maus-Myelomzelle eine Sp2/0-Agl 4-Myelomzelle ist.

21. Verfahren nach einem der Ansprüche 1 bis 13, wobei das Antigen ein Prionprotein-Peptid ist.

22. Verfahren nach Anspruch 21, wobei das Konjugieren chemisch unter Verwendung von Glutaraldehyd erfolgt.

23. Verfahren nach Anspruch 21, wobei das Prionprotein-Peptid SEQ ID NO: 6 ist.

24. Verfahren nach Anspruch 21, wobei das Prionprotein-Peptid SEQ ID NO: 7 ist.

25. Verfahren nach Anspruch 21, wobei das Prionprotein-Peptid SEQ ID NO: 9 ist.

26. Verfahren nach einem der Ansprüche 1 bis 13, wobei das Antigen Hyaluronsäure ist.

27. Verfahren nach einem der Ansprüche 1 bis 13, wobei das Antigen Matrix-Metalloprotease 3 ist.

28. Verfahren nach Anspruch 27, wobei das Konjugieren chemisch unter Verwendung von Glutaraldehyd erfolgt.

29. Zusammensetzung, hergestellt durch das Verfahren nach einem der Ansprüche 16 bis 18 und 23 bis 25.

30. Verfahren zum Verwenden monoklonaler Antikörper, die für E7-Onkoprotein spezifisch sind, hergestellt durch das Verfahren nach einem der Ansprüche 16 bis 18, für die Detektion von zervikaler intraepithelialer Neoplasie, welches folgendes umfaßt:
Screenen einer Probe von zervikalen Epithelzellen hinsichtlich des Vorliegens von E7-Onkoprotein.

31. Verfahren nach Anspruch 30, wobei das Verfahren zum Screenen hinsichtlich Vorliegen von E7-Onkoprotein aus der Gruppe ausgewählt ist, bestehend aus Radioimmuntest, heterologem Enzym-Immuntest, "Sandwich"-Immuntest, immunradiometrischem Test, Geldiffusions-Präzipitations-Reaktion, Immundiffusionstest, *in situ*-Immuntest, Western Blot, Präzipitationsreaktion, Agglutinationstest, Komplementbindungstest, Immunfluoreszenztest, Protein A-Test, Virus-Visualisierungstest, Test auf Modulation der biologischen Aktivität und Immunelektroforesetest.

32. Verfahren nach Anspruch 30, wobei das E7-Onkoprotein mit 0,05 ng/ml oder mehr vorliegt.

33. Verfahren nach Anspruch 30, wobei die monoklonalen Antikörper aus wenigstens zwei Immunglobulinisotypen bestehen.

34. Verfahren nach Anspruch 33, wobei ein Immunglobulinisotyp IgG2a ist.

35. Verfahren nach Anspruch 33, wobei ein Immunglobulinisotyp IgG2b ist.

36. Verfahren nach Anspruch 33, wobei ein Immunglobulinisotyp Spezifität für eine andere Antigendeterminante besitzt als der zweite Immunglobulinisotyp.

37. Kit zum Feststellen, ob bei einem Subjekt das Risiko besteht, daß es zervikale intraepitheliale Neoplasie entwickelt, wobei das Kit folgendes umfaßt:
a. wenigstens ein Reagens, welches E7-Onkoprotein spezifisch detektiert, wobei das Reagens der monoklonale Antikörper ist, der durch das Verfahren nach einem der Ansprüche 16 bis 18 hergestellt ist, und
b. Anweisungen zum Feststellen, daß bei dem Subjekt ein erhöhtes Risiko besteht, daß es zervikale intraepitheliale Neoplasie entwickelt.

38. Kit nach Anspruch 37, wobei das Reagens die monoklonalen Antikörper nach Anspruch 29 sind.

39. Kit zum Feststellen, ob bei einem Subjekt das Risiko besteht, daß es spongiforme Enzephalopathie entwickelt, wobei das Kit folgendes umfaßt:
a. wenigstens ein Reagens, welches Prionprotein spezifisch detektiert, wobei das Reagens die monoklonalen Antikörper sind, die durch das Verfahren nach einem der Ansprüche 23 bis 25 hergestellt sind, und
b. Anweisungen zum Feststellen, daß bei dem Subjekt ein erhöhtes Risiko besteht, daß es spongiforme Enzephalopathie entwickelt.

## Revendications

1. Procédé pour la production d'anticorps monoclonaux spécifiques d'un antigène de faible immunogénicité, comprenant les étapes consistant à :
a. conjuguer l'antigène chimiquement à une molécule du support, ladite molécule de support étant une protéine de choc thermique ;
b. immuniser un animal non humain avec l'antigène conjugué ;
c. recueillir des lymphocytes B à partir de l'animal ;
d. créer un hybridome à partir des lymphocytes B recueillis ; et
e. soumettre les hybridomes à une sélection pour la spécificité vis-à-vis de l'antigène naturel.

2. Procédé suivant la revendication 1, dans lequel la molécule de support est la molécule HSP70.

3. Procédé suivant la revendication 1, dans lequel l'animal a un système immunitaire intact.

4. Procédé suivant la revendication 1, dans lequel l'animal est un mammifère.

5. Procédé suivant la revendication 1, dans lequel les lymphocytes B sont recueillis à partir d'ascites.

6. Procédé suivant la revendication 1, dans lequel les lymphocytes B sont recueillis à partir de ganglions lymphatiques.

7. Procédé suivant la revendication 1, dans lequel les lymphocytes B sont recueillis à partir du sang.

8. Procédé suivant la revendication 1, dans lequel les lymphocytes B sont recueillis à partir de la rate.

9. Procédé suivant la revendication 1, dans lequel l'hybridome est créé en utilisant une cellule immortelle de souris.

10. Procédé suivant la revendication 9, dans lequel la cellule immortelle de souris est une cellule de myélome de souris.

11. Procédé suivant la revendication 1, dans lequel l'hybridome est créé en utilisant une cellule immortelle humaine.

12. Procédé suivant la revendication 1, dans lequel l'hybridome est créé en utilisant une cellule immortelle de rat.

13. Procédé suivant la revendication 1, dans lequel la sélection pour la spécificité est effectuée par un procédé choisi dans le groupe consistant en une analyse radio-immunologique, une analyse par immunosorbant lié à une enzyme, une analyse immunologique "en sandwich", une analyse immunoradiométrique, une réaction de précipitation par diffusion en gel, une analyse d'immunodiffusion, une analyse immunologique in situ, une analyse de transfert d'empreinte par la méthode Western, une réaction de précipitation, une analyse d'agglutination, une analyse de fixation du complément, une analyse d'immunofluorescence, une analyse utilisant la protéine A, une analyse de visualisation de virus, une analyse de modulation d'activité biologique et une analyse d'immunoélectrophorèse.

14. Procédé suivant l'une quelconque des revendications 1 à 13, dans lequel l'antigène est l'oncoprotéine E7.

15. Procédé suivant la revendication 14, dans lequel la conjugaison chimique comprend :
a. la création d'un plasmide ayant une séquence de nucléotides codant pour l'oncoprotéine E7 et une séquence de nucléotides codant pour HSP70 ; et
b. la transfection d'une cellule hôte avec le plasmide, ladite cellule hôte effectuant la transcription des séquences de nucléotides en l'oncoprotéine E7 conjuguée.

16. Procédé suivant la revendication 15, dans lequel la séquence de nucléotides codant pour l'oncoprotéine E7 est la SEQ ID N° 1.

17. Procédé suivant la revendication 15, dans lequel la séquence de nucléotides codant pour l'oncoprotéine E7 est la SEQ ID N° 3.

18. Procédé suivant la revendication 15, dans lequel la séquence de nucléotides codant pour HSP70 est la SEQ ID N° 5.

19. Procédé suivant la revendication 15, dans lequel la cellule hôte est E. coli.

20. Procédé suivant la revendication 14, dans lequel la cellule de myélome de souris est une cellule de myélome Sp2/0-Ag14.

21. Procédé suivant l'une quelconque des revendications 1 à 13, dans lequel l'antigène est un peptide de protéine Prion.

22. Procédé suivant la revendication 21, dans lequel la conjugaison est effectuée chimiquement en utilisant du glutaraldéhyde.

23. Procédé suivant la revendication 21, dans lequel le peptide de protéine Prion est le peptide de SEQ ID N° 6.

24. Procédé suivant la revendication 21, dans lequel le peptide de protéine Prion est le peptide de SEQ ID N° 7.

25. Procédé suivant la revendication 21, dans lequel le peptide de protéine Prion est le peptide de SEQ ID N° 9.

26. Procédé suivant l'une quelconque des revendications 1 à 13, dans lequel l'antigène est l'acide hyaluronique.

27. Procédé suivant l'une quelconque des revendications 1 à 13, dans lequel l'antigène est la métalloprotéase de matrice 3.

28. Procédé suivant la revendication 27, dans lequel la conjugaison est effectuée chimiquement en utilisant du glutaraldéhyde.

29. Composition produite par le procédé de l'une quelconque des revendications 16-18 et 23-25.

30. Méthode d'utilisation d'anticorps monoclonaux spécifiques de l'oncoprotéine E7 préparés par le procédé de l'une quelconque des revendications 16 à 18 pour la détection d'une néoplasie intraépithéliale du col, comprenant l'étape consistant à soumettre un échantillon de cellules épithéliales du col à un dépistage de la présence de l'oncoprotéine E7.

31. Méthode suivant la revendication 30, ladite méthode de dépistage de la présence de l'oncoprotéine E7 étant choisie dans le groupe consistant en une analyse radio-immunologique, une analyse par immunosorbant lié à une enzyme, une analyse immunologique "en sandwich", une analyse immunoradiométrique, une réaction de précipitation par diffusion en gel, une analyse d'immunodiffusion, une analyse immunologique in situ, une analyse de transfert d'empreinte par la méthode Western, une réaction de précipitation, une analyse d'agglutination, une analyse de fixation du complément, une analyse d'immunofluorescence, une analyse utilisant la protéine A, une analyse de visualisation de virus, une analyse de modulation d'activité biologique et une analyse d'immunoélectrophorèse.

32. Méthode suivant la revendication 30, dans laquelle la présence de l'oncoprotéine E7 est égale ou supérieure à 0,05 ng/ml.

33. Méthode suivant la revendication 30, dans laquelle les anticorps monoclonaux comprennent au moins deux isotypes d'immunoglobuline.

34. Méthode suivant la revendication 33, dans laquelle un isotype d'immunoglobuline est l'isotype IgG2a.

35. Méthode suivant la revendication 33, dans laquelle un isotype d'immunoglobuline est l'isotype IgG2b.

36. Méthode suivant la revendication 33, dans laquelle un isotype d'immunoglobuline présente une spécificité pour un déterminant antigénique différent, par rapport au second isotype d'immunoglobuline.

37. Kit pour déterminer si un sujet présente un risque de développer une néoplasie intraépithéliale du col, comprenant :
a. au moins un réactif qui détecte spécifiquement l'oncoprotéine E7, ledit réactif étant l'anticorps monoclonal préparé par le procédé de l'une quelconque des revendications 16 à 18 ; et
b. des instructions pour déterminer que le sujet présente un risque accru de développer une néoplasie intraépithéliale du col.

38. Kit suivant la revendication 37, dans lequel le réactif consiste en les anticorps monoclonaux de la revendication 29.

39. Kit pour déterminer si un sujet présente un risque de développer une encéphalopathie spongiforme, comprenant :
a. au moins un réactif qui détecte spécifiquement la protéine Prion, ledit réactif consistant en les anticorps monoclonaux préparés par le procédé de l'une quelconque des revendications 23 à 25 ; et
b. des instructions pour déterminer que le sujet présente un risque accru de développer une encéphalopathie spongiforme.
